# EUROPEAN PATENT APPLICATION

(11) **EP 2 725 360 A1**
(43) Date of publication of application: **30.04.2014**
(21) Application number: 12382411.2
(22) Date of filing: 24.10.2012
(51) Int. Cl.: G01N 33/68

(54) **Biomarkers for the prognosis of ischemic stroke**

(71) Applicant: Fundació Hospital Universitari Vall d'Hebron - Institut de Recerca, 08035 Barcelona (ES)
(72) Inventor: Garcia-Berrocoso, Teresa, E-08016 Barcelona (ES); Montaner Villalonga, Joan, E-08037 Barcelona (ES)
(74) Representative: ABG Patentes, S.L.

(57) **Abstract**

The invention relates to methods for determining the clinical outcome of a patient suffering ischemic stroke, for designing an individual therapy and for diagnosing a silent cerebrovascular disease comprising determining the levels of a marker selected from gelsolin, CysA, DPYSL2 or a combination thereof, the altered expression of which in relation to a reference value allows determining the clinical outcome of a patient suffering ischemic stroke, designing for designing an individual therapy or diagnosing a silent cerebrovascular disease. Furthermore, the invention relates to a kit comprising a reagent for detecting the level of a marker selected from gelsolin, CysA, DPYSL2 or a combination thereof and to the use of the said kit in the methods of the invention.

## Description

### TECHNICAL FIELD OF THE INVENTION

The invention relates to the field of diagnostic, in particular to a method of predicting the clinical outcome of patients suffering from ischemic stroke based on the expression of certain biomarkers in a sample from said patients.

### BACKGROUND OF THE INVENTION

In an ischemic stroke, blood supply to part of the brain is decreased, leading to dysfunction of the brain tissue in that area. There are four reasons why this might happen, thrombosis (obstruction of a blood vessel by a blood clot forming locally), embolism (obstruction due to an embolus from elsewhere in the body, see below), systemic hypoperfusion (general decrease in blood supply, e.g., in shock) or venous thrombosis. Stroke without an obvious explanation is termed "cryptogenic"; this constitutes 30-40% of all ischemic strokes. Moreover, the term "silent brain infarcts (SBIs)" is widely used to describe cerebral infarcts seen on brain CT or MRI without any corresponding stroke episode and SBIs have been found to be highly prevalent and associated with adverse health outcomes.

There are various classification systems for ischemic stroke. The Oxford Community Stroke Project classification (OCSP, also known as the Bamford or Oxford classification) relies primarily on the initial symptoms; based on the extent of the symptoms, the stroke episode is classified as total anterior circulation infarct (TACI), partial anterior circulation infarct (PACI), lacunar infarct (LACI) or posterior circulation infarct (POCI). These four entities predict the extent of the stroke, the area of the brain affected, the underlying cause, and the prognosis. The TOAST (Trial of Org 10172 in Acute Stroke Treatment) classification is based on clinical symptoms as well as results of further investigations and denotes five subtypes of ischemic stroke: 1) large-artery atherosclerosis, 2) cardioembolism, 3) small-vessel occlusion, 4) stroke of other determined etiology, and 5) stroke of undetermined etiology.

Ischemic stroke is the third cause of death and the major cause of disability worldwide. About 20% of people who have a stroke die in the hospital, being the proportion higher among older people.

Many stroke survivors recover functional independence after a stroke: about 10% of people who have an ischemic stroke recover almost all normal function, and about 25% recover most of it. Regrettably 25% are left with a minor disability and 40% experience moderate to severe impairments requiring special care, and about 10% require care in a nursing home or other long-term care facility. Some people are physically and mentally devastated and unable to move, speak, or eat normally. About 25% of people who recover from a stroke have another stroke within 5 years and subsequent strokes impair function further.

During the first few days after an ischemic stroke, doctors usually cannot predict whether a person will improve or worse. Younger people and people who start improving quickly are likely to recover more fully. About 50% of people with one-sided paralysis and most of those with less severe symptoms recover some function by the time they leave the hospital, and they can eventually take care of their basic needs. They can think clearly and walk adequately, although use of the affected arm or leg may be limited. Most impairments still present after 12 months are permanent.

The National Institutes of Health's stroke scale (NIHSS) helps predict the severity and outcome of a stroke by scoring 11 factors (levels of consciousness, gaze, visual fields, facial movement, motor functions in the arm and leg, coordination, sensory loss, problems with language, inability to articulate, and attention). Those patients with ischemic strokes who score less than 10 have a favorable outlook after a year, while only 4 - 16% of patients do well if their score is more than 20. The prognosis of stroke patients is still based only on clinical and neuroimaging data, which is not a rapid and accessible tool.

What a clinician requires of a good prognostic model is that it be based on a representative sample of patients, make clinical sense, be accurate and simple. However, studies often differ in their selection of patients, *e.g* community based, hospital based, stroke unit based or restricted to patients under 75 years.

Predicting outcome in individuals, however, remains difficult, because prognostic studies examining associations between clinical signs or syndromes and outcome differ in patient selection, timing and choice of neurological assessments and outcome measures. Some use single signs (*e.g* level of consciousness or incontinence), others use combinations of signs (*e.g* visual neglect, level of consciousness,hemiplegia). Some use neurological scales or have investigated neuroimaging (CT scan, NMR, SPECT, PET).

Moreover widely used tools for prediction the outcome of a disease are fundamentally limited in that they do not account for effects of withdrawal of care and are not designed to predict functional recovery.

In fact these limitations imply that we are not using such prediction tools in routine practice and are not giving objective prognostic information (i.e. 85% probability of poor outcome) in daily practice to our stroke patients.

Blood biomarkers might add information to improve the prediction of stroke outcome. However, no known biomarker or biomarkers panel exists nowadays that provides a higher sensitivity and specificity than clinical predictor models (Whiteley W, PLoS Med., 2009, 6(9), e1000145). New statistical tools are being employed to assess this improvement, as net reclassification improvement (NRI) or integrated discrimination improvement (IDI) indexes (Pencina et al, Stat. Med., 2008, 27, 157-72; Whiteley W et al, PLoS Med., 2009, 6(9), e1000145).

The ability to discern stroke patients who are going to develop medical complications (as infections, hemorrhagic transformation or edema) or functional disabilities from those who are not would allow the optimization of the admission of patients to the specialized stroke units. The concept of stroke unit has allowed to reduce the in-hospital lethality and to improve the functional outcome of stroke patients by means of specialized nursing care and appropriate monitoring of the patients, albeit with increased costs. Thus it is very important to correctly identify those patients in need of therapies in a specialized stroke unit.

Therefore, there is a need in the art for further methods useful for predicting the clinical outcome of a patient suffering from stroke.

### SUMMARY OF THE INVENTION

In an aspect, the invention relates to an *in vitro* method for determining the clinical outcome of a patient suffering ischemic stroke, comprising
a) determining the level of a marker selected from gelsolin, CysA, DPYSL2 or a combination thereof, in a sample from said patient, and
b) comparing said level with a reference value for each marker,
   wherein if the level of gelsolin or CysA in a sample from said patient is higher than a reference value for each marker and/or if the level of DPYSL2 in a sample from said patient is lower than a reference value for said marker is indicative of a negative clinical outcome
   or
   wherein if the level of gelsolin or CysA in a sample from said patient is lower than a reference value for each marker or if the level of DPYSL2 in a sample from said patient is higher than a reference value for said marker is indicative of a positive clinical outcome.

In another aspect, the invention relates to an *in vitro* method for designing an individual therapy for a subject suffering ischemic stroke comprising
a) determining the level of a marker selected from gelsolin, CysA, DPYSL2 or a combination thereof, in a sample from said patient, and
b) comparing said level with a reference value for each marker,
wherein if the level of gelsolin or CysA in a sample from said patient is higher than a reference value for each marker and/or if the level of DPYSL2 in a sample from said patient is lower than the reference value, is indicative that the patient is a candidate for a therapy in a specialized stroke unit.

In another aspect, the invention relates to an *in vitro* method for diagnosing a silent cerebrovascular disease comprising:
a) determining the level of a marker selected from gelsolin or DPYSL2 or a combination thereof, in a sample from said patient, and
b) comparing said level with a reference value for each marker,
wherein if the level of DPYSL2 in a sample from said patient is higher than a reference value for said marker or if the level of gelsolin in a sample from said patient is lower than a reference value for said marker is indicative that the patient has a silent cerebrovascular disease.

In another aspect, the invention relates to a kit comprising a reagent for detecting the level of a marker selected from gelsolin, CysA, DPYSL2 or a combination thereof.

In another aspect, the invention relates to the use of a kit of the invention for determining the clinical outcome of a patient suffering ischemic stroke, for designing an individual therapy for a subject suffering ischemic stroke or for diagnosing a silent cerebrovascular disease.

### BRIEF DESCRIPTION OF THE DRAWINGS

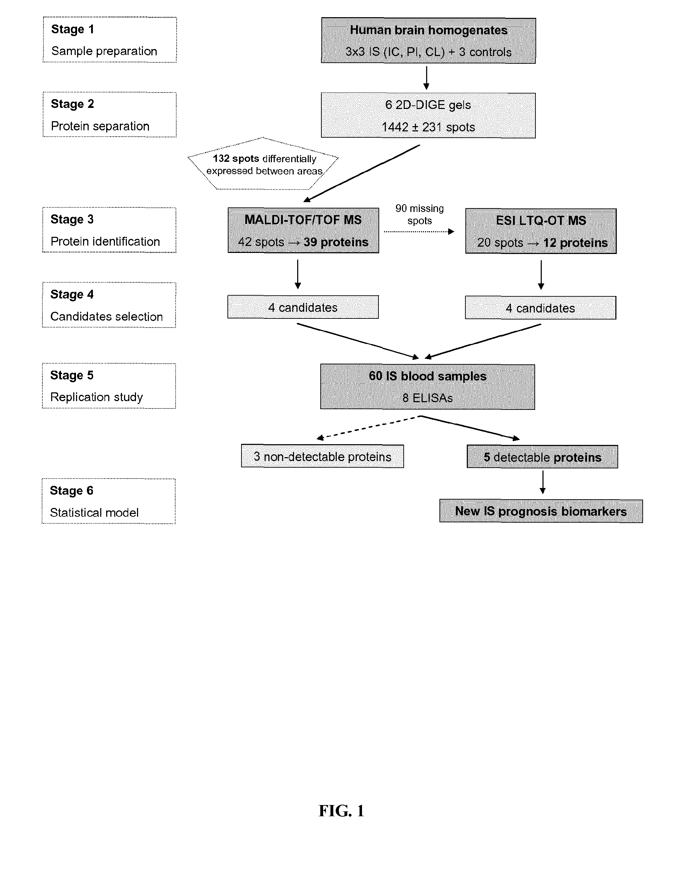

**Figure 1****.** Description of the workflow.

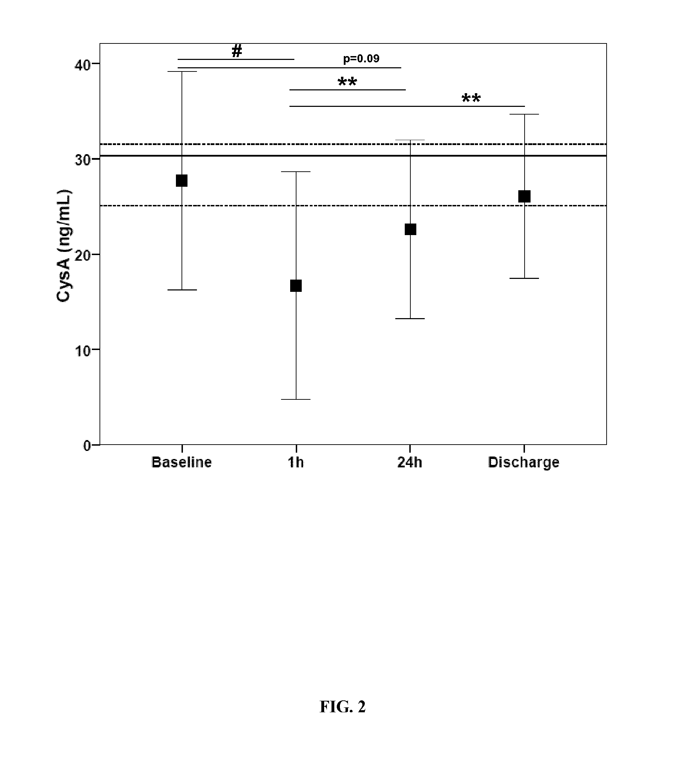

**Figure 2****.** Blood temporal profile of CysA. Error bars represent mean ± 1SD for CysA plasma levels in ischemic stroke patients (N=11). Continuous line represents median and discontinuous lines represent lower and upper quartiles of CysA plasma levels in healthy controls (N=5; only as reference values). # p<0.001, ** p < 0.01.

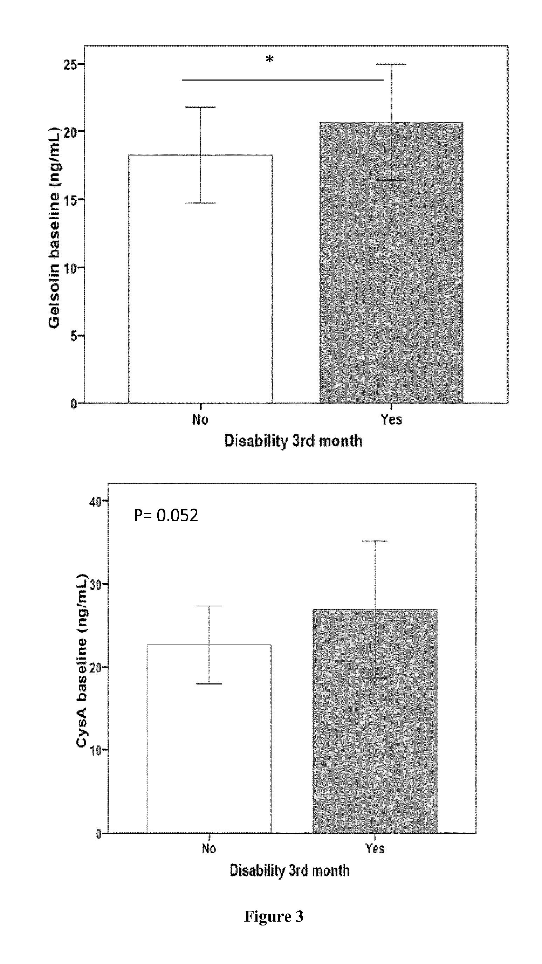

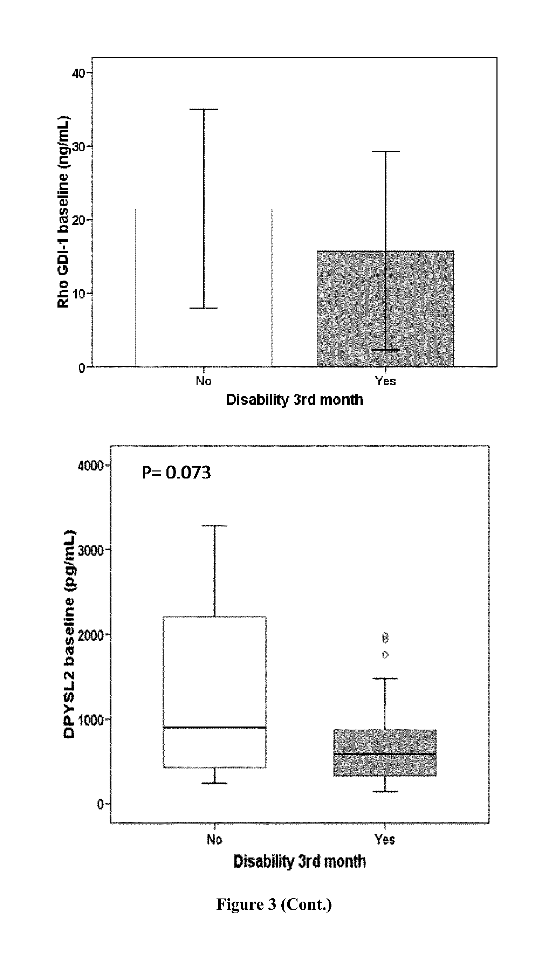

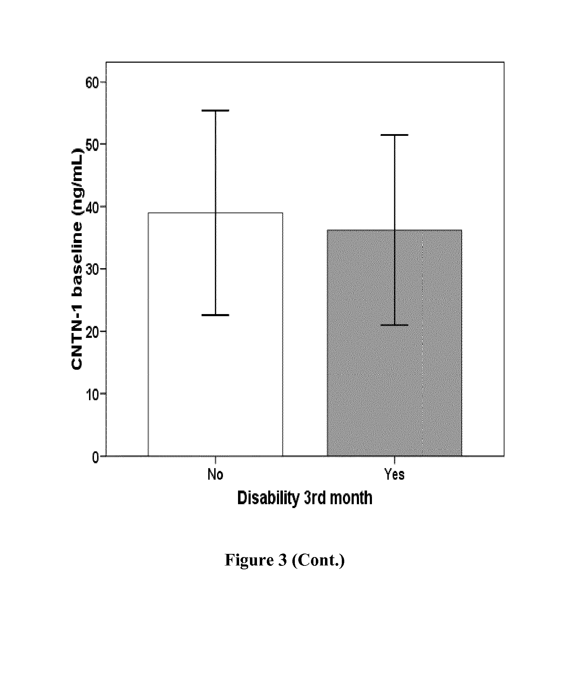

**Figure 3****.** Blood level of biomarker candidates regarding long-term outcome. Graphs represent baseline biomarkers levels in relation to disability at 3rd month after ischemic stroke (N=60 patients). Bar graphs represent mean with error bars indicating 1SD. Box-plot represents median and interquartile range. * p<0.05.

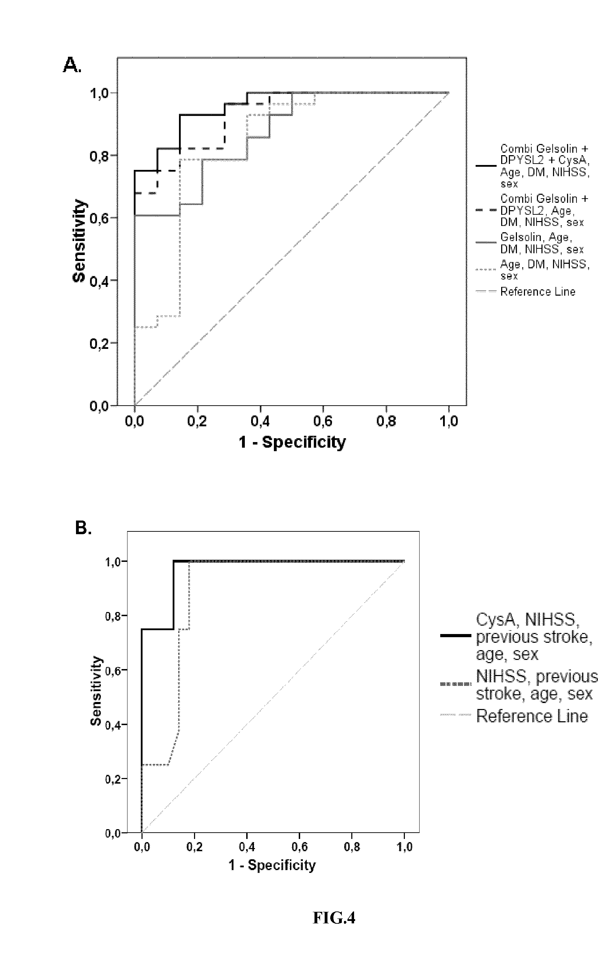

**Figure 4****.** ROC curves of the predictive models for disability at 3rd month (A) and in-hospital mortality (B). Comparison between only clinical data (discontinuous line) and clinical data plus biomarkers (continuous line). Biomarkers were added to clinical model using cut-off point: Gelsolin>19.87 ng/mL, DPYSL2<2095.69 pg/mL, CysA>31.76 ng/mL (for disability at 3rd month) or CysA>34.35 ng/mL (for in-hospital mortality). DM, diabetes mellitus; NIHSS, National Institutes of Health Stroke Scale.

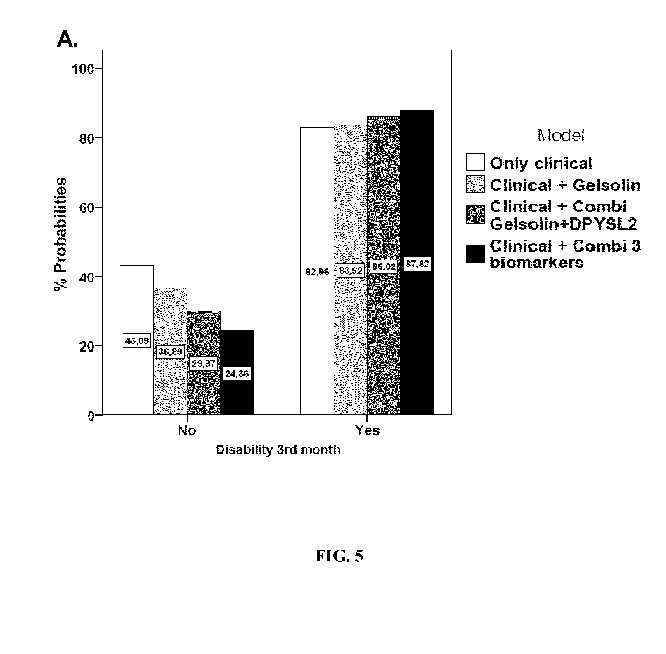

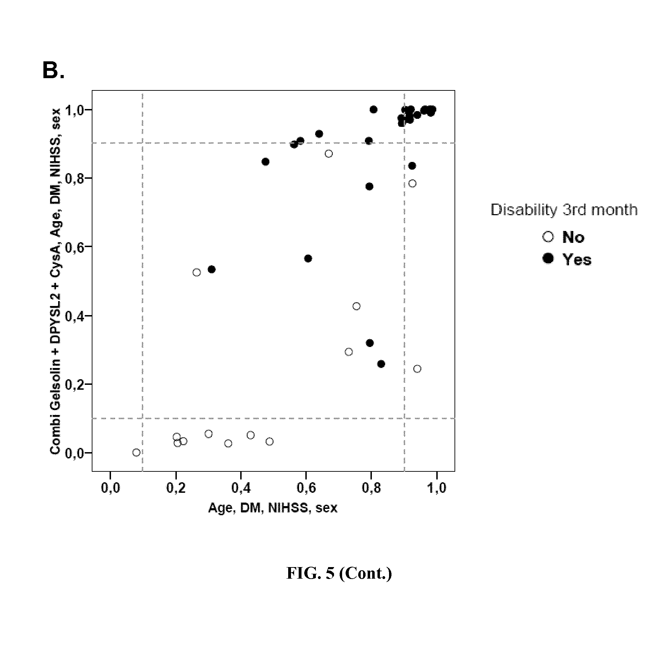

**Figure 5****.** Prediction of functional outcome at 3rd month regarding the model. (A) Bars show the mean of percentage of probability of each model to predict disability at 3rd month. The more biomarkers in the model, the best prediction, mainly reducing false positives (non-disabled). (B) Scatter dot graph showing probability for only clinical model (x axis) and model with the combination of 3 biomarkers (y axis); empty dots (○) represent patients not disabled at 3rd month and full dots (●), patients disabled at 3rd month. Discontinuous lines represent pre-specified risk categories (10% and 90%). The model with 3 biomarkers predicts better the disability of patients (dots in categories >90% or <10% only when biomarkers were used). Biomarkers were added to clinical model using cut-off point: Gelsolin>19.87 ng/mL, DPYSL2<2095.69 pg/mL, CysA>31.76 ng/mL.

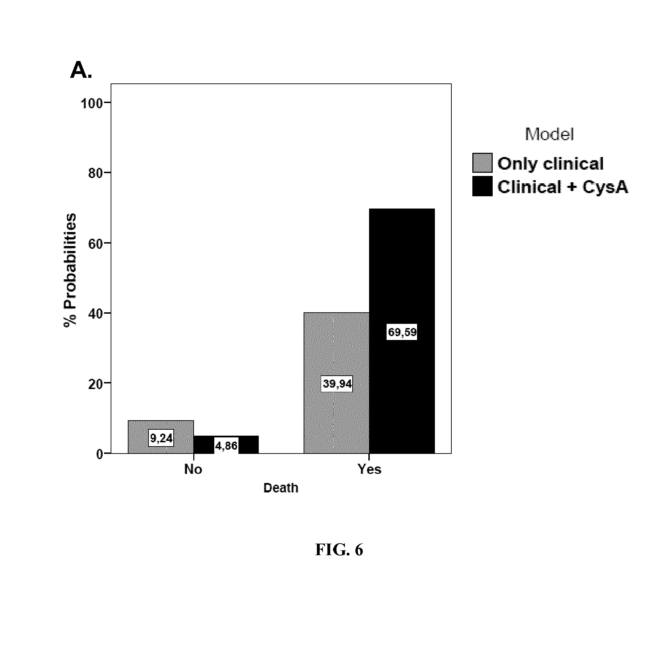

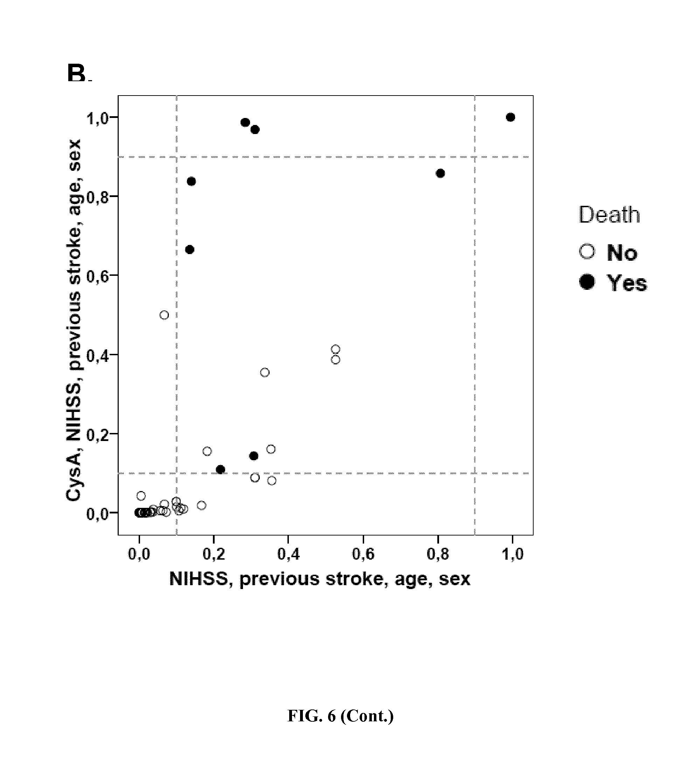

**Figure 6****.** Prediction of in-hospital mortality regarding the model. (A) Bars show the mean of percentage of probability of each model to predict death. The presence of CysA in the model allowed a better prediction of events (deaths) and also reduced false positives (survivors). (B) Scatter dot graph showing probability for only clinical model (x axis) and model with CysA (y axis); empty dots (o) represent survivors and full dots (●), exitus. Discontinuous lines represent pre-specified risk categories (10% and 90%).
The model with CysA predicts much better the exitus (dots in categories >90% or <10% only when CysA was used). CysA was added to clinical model using cut-off point: CysA>34.35 ng/mL.

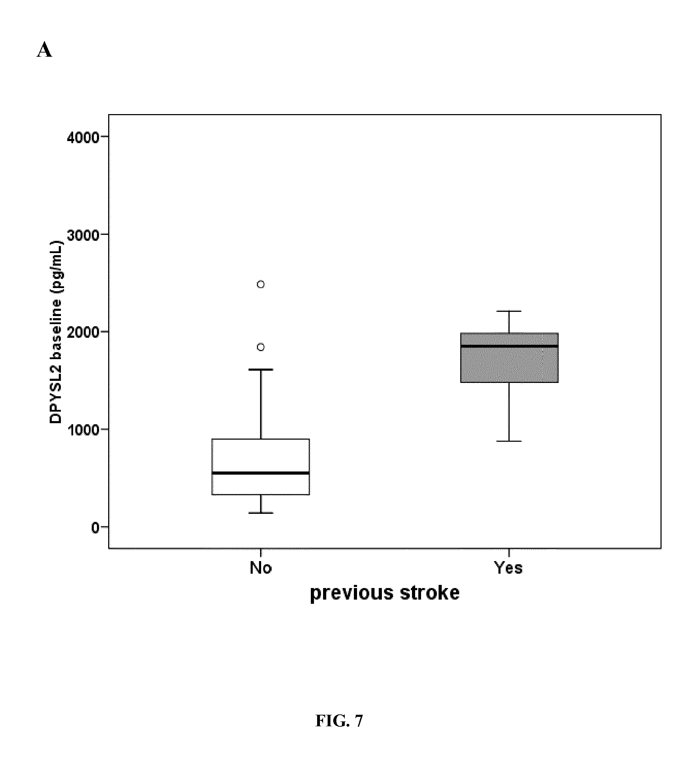

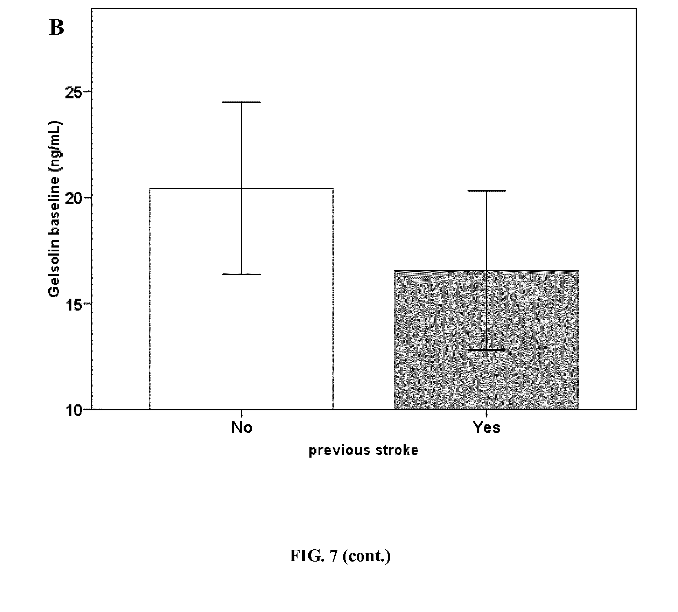

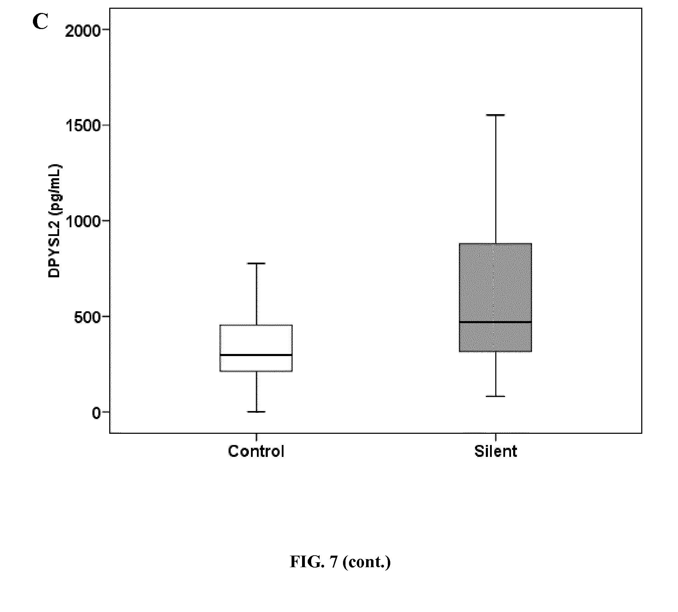

**Figure 7****.** Blood level of biomarker candidates regarding subclinical cerebral infarcts. (A) Graphs represent baseline biomarkers levels in relation to the presence of previous stroke when attending the hospital for the index stroke event (N=60 stroke patients). (B) Graph represent biomarker level in relation to unknown asymptomatic silent cerebral infarct among patients free of stroke (N=36 subjects). Bar graph represents mean with error bar indicating 1SD and box-plots represent median and interquartile range.* p<0.05, **** p<0.01.

### DETAILED DESCRIPTION OF THE INVENTION

The inventors of the present invention have identified biomarkers which can be used to determine the degree of brain damage during ischemic stroke. These markers can provide an estimation of the clinical outcome of a stroke patient (see examples 4 and 5 of the present invention). The biomarkers identified in the present invention include, particularly, gelsolin, CysA and DPYSL2.

### Method for determining the clinical outcome of a patient suffering ischemic stroke

In an aspect, the invention relates to an *in vitro* method for determining the clinical outcome of a patient suffering ischemic stroke (first method of the invention), comprising
a) determining the level of a marker selected from gelsolin, CysA, DPYSL2 or a combination thereof, in a sample from said patient, and
b) comparing said level with a reference value for each marker,
wherein if the level of gelsolin or CysA in a sample from said patient is higher than a reference value for each marker and/or if the level of DPYSL2 in a sample from said patient is lower than a reference value for said marker is indicative of a negative clinical outcome
or
wherein if the level of gelsolin or CysA in a sample from said patient is lower than a reference value for each marker or if the level of DPYSL2 in a sample from said patient is higher than a reference value for said marker is indicative of a positive clinical outcome.

The term "determining", as used herein, relates to the determination of any parameter that can be useful in determining the evolution of a patient. As will be understood by those skilled in the art, the prediction of the outcome, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

In the present invention "clinical outcome" is understood as the expected course of a disease. It denotes the doctor's prediction of how a subject's disease will progress, and whether there is chance of recovery, disability or mortality.

A patient suffering ischemic stroke is considered to have "negative clinical outcome" or bad prognosis if the patient die after stroke or if he cannot make basic activities of daily living (BADL) which includes feeding; grooming; dressing; bathing; toileting, including sphincter control; and mobility, including transferring from place to place. Independence in BADL could enable the stroke patient to live at home with help from family or community providers for meals and other household tasks as needed.

In a preferred embodiment, the negative clinical outcome determined by the first method of the invention is a functionally disability at third month after stroke.

The term "functional disability" represents the social and societal consequences of functional limitations. It is defined by a patient's inability to perform activities of daily living and maintain social and family relationships, to continue in a vocation, or to pursue leisure activities. A functional disability limits a person's ability to perform physical activities, need long-term care, use assistive devices or technology. In the present invention functional outcome was evaluated at third month after the event by means of the modified Rankin Scale (mRS); disability of patients was considered when mRS score is higher than 2.

The "modified Rankin Scale" refers to a commonly used scale for measuring the degree of disability or dependence in the daily activities of people who have suffered a stroke, and it has become the most widely used clinical outcome measure for stroke clinical trials. The scale runs from 0-6, running from perfect health without symptoms to death.
0. No symptoms.
1. No significant disability. Able to carry out all usual activities, despite some symptoms.
2. Slight disability. Able to look after own affairs without assistance, but unable to carry out all previous activities.
3. Moderate disability. Requires some help, but able to walk unassisted.
4. Moderately severe disability. Unable to attend to own bodily needs without assistance, and unable to walk unassisted.
5. Severe disability. Requires constant nursing care and attention, bedridden, incontinent.
6. Death.

In another preferred embodiment, the negative clinical outcome determined by the first method of the invention is in-hospital mortality.

The term "in hospital mortality" was defined as death occurring during the hospital stay.

A patient suffering ischemic stroke is considered to have "positive clinical outcome" or good prognosis is the patient after stroke can make basic activities of daily living and more complex activities of daily living called instrumental activities of daily living (IADL). These tasks are performed to maintain independence in the home and community and include shopping, using transportation, telephoning, preparing meals, handling finances, and maintaining a household. Independence in these activities enables the stroke patient to be discharged to home without being dependent on others.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race.

The term "ischemic stroke" refers to the physical blockage of blood flow to an area of the brain, causing brain cells in the area to die.

In a first step, the method for determining the clinical outcome of a patient suffering ischemic stroke according to the invention comprises determining the level of a marker selected from gelsolin, CysA, DPYSL2 or a combination thereof, in a sample from a patient.

The term "gelsolin", as used herein, refers to an actin binding protein of 82-kD with six homologous subdomains, referred to as S1-S6, which acts as a key regulator of actin filament assembly and disassembly. The complete protein sequence for human gelsolin has the UniProt accession number P06396 (July 11^{th}, 2012).

The term "CysA", as used herein, refers to Cystatin A, CSTA or stefin A, protection of cytosolic and cytoskeleton proteins from degradation by cysteine proteases accidentally released from lysosomes. The complete protein sequence for human CysA has the UniProt accession number P01040 (July 11^{th}, 2012).

The term"DPYSL2", as used herein, refers to a dihydropyrimidinase-related protein 2 or Collapsin response mediator protein 2, a protein that promotes microtubule assembly and is required for Sema3A-mediated growth cone collapse, and also plays a role in synaptic signaling through interactions with calcium channels. The complete protein sequence for human dihydropyrimidinase-related protein 2 has the UniProt accession number Q16555 (July 11^{th}, 2012).

In a preferred embodiment, the methods of the invention comprise determining the levels of one of the markers selected from CysA, gelsolin or DPYSL2. In another preferred embodiment, the methods of the invention comprise determining the levels of a combination of any two markers selected form the group consisting of CysA and gelsolin, CysA and DPYSL2, gelsolin and DPYSL2. In another preferred embodiment the invention comprises determining the levels of gelsolin, CysA and DPYSL2.

In one embodiment, when the levels of gelsolin or CysA are determined, the determination is carried out in plasma. In another embodiment, when the levels of DPYSL2 are determined, the determination is carried out in serum.

In order to carry out the method of the invention, a sample is obtained from the subject under study.

The term "sample" as used herein, relates to any sample which can be obtained from the patient. The present method can be applied to any type of biological sample from a patient, such as a biopsy sample, tissue, cell or biofluid (plasma, serum, saliva, semen, sputum, cerebral spinal fluid (CSF), tears, mucus, sweat, milk, brain extracts and the like). In a preferred embodiment the sample is a biofluid. In a more preferred embodiment the biofluid is blood, plasma, serum, saliva, urine or cerebrospinal fluid.

As the person skilled in the art understands, the expression levels of gelsolin, CysA and DPYSL2 can be determined by measuring the levels of mRNA encoded by the corresponding genes or by measuring the levels of proteins encoded by said genes, and the levels of variants thereof.

By way of a non-limiting illustration, the expression levels are determined by means of the quantification of the levels of mRNA encoded by said genes. The latter can be quantified by means of using conventional methods, for example, methods comprising the amplification of mRNA and the quantification of the amplification product of said mRNA, such as electrophoresis and staining, or alternatively, by means of Northern blot and the use of suitable probes, Northern blot and use of specific probes of the mRNA of the genes of interest or of their corresponding cDNA/cRNA, mapping with the S1 nuclease, RT-PCR, hybridization, microarrays, etc. Similarly, the levels of the cDNA/cRNA corresponding to said mRNA encoded by the marker genes can also be quantified by means of using conventional techniques; in this event, the method of the invention includes a step of synthesis of the corresponding cDNA by means of reverse transcription (RT) of the corresponding mRNA followed by the synthesis (RNA polymerase) and amplification of the cRNA complementary to said cDNA. Conventional methods of quantifying the expression levels can be found, for example, in Sambrook et al., 2001 "Molecular cloning: to Laboratory Manual", 3rd ed., Cold Spring Harbor Laboratory Press, N.Y., Vol. 1-3.

In order to normalize the values of mRNA expression among the different samples, it is possible to compare the expression levels of the mRNA of interest in the test samples with the expression of a control RNA. A "control RNA" as used herein, relates to RNA whose expression levels do not change or change only in limited amounts. Preferably, the control RNA is mRNA derived from housekeeping genes and which code for proteins which are constitutively expressed and carry out essential cellular functions. Preferred housekeeping genes for use in the present invention include 18-S ribosomal protein, β-2-microglobulin, ubiquitin, cyclophilin, GAPDH, PSMB4, tubulin and β-actin.

Alternatively, it is also possible to determine the expression levels of the marker genes by means of the determination of the expression levels of the proteins encoded by said genes, since if the expression of genes is increased, an increase of the amount of corresponding protein should occur and if the expression of genes is decreased, a decrease of the amount of corresponding protein should occur.

The determination of the expression levels of the proteins can be carried out by immunological techniques such as ELISA, Western Blot or immunofluorescence. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry or fluorometry.

On the other hand, the determination of the protein expression levels can be carried out by constructing a tissue microarray (TMA) containing the subject samples assembled, and determining the expression levels of the proteins by techniques well known in the state of the art.

In a preferred embodiment the determination of the levels of the markers are determined by immunological technique. In a more preferred embodiment, the immunological technique is ELISA.

When an immunological method is used, any antibody or reagent known to bind with high affinity to the target proteins can be used for detecting the amount of target proteins. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

As previously cited, the expression levels of the gelsolin, CysA and DPYSL2 can be determined by measuring both the levels of protein, and the levels of variants thereof, such as fragments, analogues and/or derivatives.

Variants of the gelsolin, CysA and DPYSL2 may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the protein is an alternative splice variant of the proteins of the present invention and/or (iv) fragments of the proteins. The fragments include proteins generated via proteolytic cleavage (including multi-site proteolysis) of an original sequence. Variants are deemed to be within the scope of those skilled in the art from the teaching herein.

As known in the art the "similarity" between two proteins is determined by comparing the amino acid sequence and its conserved amino acid substitutes of one protein to a sequence of a second protein. Variants according to the present invention includes amino acid sequences that are at least 60%, 65%, 70%, 72%, 74%, 76%, 78%, 80%, 90%, or 95% similar or identical to the original amino acid sequence. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be post-translationally modified. For example, post-translational modifications that fall within the scope of the present invention include signal peptide cleavage, glycosylation, acetylation, isoprenylation, proteolysis myristoylation, protein folding and proteolytic processing, etc. Additionally, the proteins may include unnatural amino acids formed by post-translational modification or by introducing unnatural amino acids during translation.

In a preferred embodiment the patient has been treated with a thrombolytic agent.

The term "thrombolytic agent" as used herein refers to a drug that is able to dissolve a clot. All thrombolytic agents are serine proteases and convert plasminogen to plasmin which breaks down the fibrinogen and fibrin and dissolves the clot. Currently available thrombolyic agents include reteplase (r-PA or Retavase), alteplase (t-PA or Activase), urokinase (Abbokinase), prourokinase, anisoylated purified streptokinase activator complex (APSAC), staphylokinase (Sak) and streptokinase. In a preferred embodiment the thrombolytic agent is plasminogen activator. In a more preferred embodiment the plasminogen activator is tissue plasminogen activator (t-PA).

The term "tissue plasminogen activator (t-PA)" as used herein refers to a serine protease found on endothelial cells that catalyzes the conversion of plasminogen to plasmin. The complete protein sequence for human t-PA has the UniProt accession number P00750 (July 11^{th}, 2012). tPA may be manufactured using recombinant biotechnology techniques, tPA created this way may be referred to as recombinant tissue plasminogen activator (rtPA). Recombinant tissue plasminogen activators (r-tPAs) include alteplase, reteplase, and tenecteplase (TNKase).

Doses of t-PA should be given within the first 3 hours of the onset of symptoms. Recommended total dose: 0.9 mg/kg (maximum dose should not exceed 90 mg) infused over 60 minutes. Load with 0.09 mg/kg (10% of the 0.9 mg/kg dose) as an intravenous bolus over 1 minute, followed by 0.81 mg/kg (90% of the 0.9 mg/kg dose) as a continuous infusion over 60 minutes. Heparin should not be started for 24 hours or more after starting alteplase for stroke. Said t-PA is given intravenously and in some cases may be given directly into an artery and should be given right away after the first symptoms of stroke start.

The second step of the method for determining the clinical outcome of a patient suffering ischemic stroke comprises comparing the level/s of the marker/s with a reference value for each marker.

The term "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

According to the present invention, the level of a biomarker is increased when the level of said biomarker in a sample is higher than a reference value. The levels of a biomarker are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more higher than the reference value.

Likewise, in the context of the present invention, the level of a biomarker is decreased when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more lower than the reference value.

According to the first method of the invention if the negative clinical outcome is a functionally disability at third month after stroke, the reference value of gelsolin is about 19.87 ng protein/ml, the reference value of DPYSL2 is about 2095.69 pg protein/ml and the reference value of CysA is about 31.76 ng protein/ml.

In another embodiment of the first method of the invention if the negative clinical outcome is in hospital mortality the reference value of CysA is about 34.35 ng protein/ml.

In another embodiment the methods of the invention further comprises determining one or more clinical data indicative of clinical outcome of a patient after suffering ischemic stroke.

The term "clinical data" as used herein refers to person demographics (age or date of birth, race and/or ethnicity), patient clinical symptoms or signs related to stroke related diseases/conditions.

In a preferred embodiment the clinical data is selected from age, NIHSS score, presence of diabetes mellitus or previous stroke and sex, wherein old age, higher NIHSS score, suffering diabetes mellitus or previous stroke, and being woman are indicative of a negative clinical outcome.

The term "old age" refers to any age after 60.

The term "NIHSS score", as used in the present invention refers to The National Institutes of Health Stroke Scale (NIHSS) score, a systematic assessment tool that provides a quantitative measure of stroke-related neurologic deficit (Adams HP Jr Neurology. 1999 Jul 13;53(1):126-31). The NIHSS was originally designed as a research tool to measure baseline data on patients in acute stroke clinical trials. Now, the scale is also widely used as a clinical assessment tool to evaluate acuity of stroke patients, determine appropriate treatment, and predict patient outcome. The NIHSS is a 15-item neurologic examination stroke scale used to evaluate the effect of acute cerebral infarction on the levels of consciousness, language, neglect, visual-field loss, extraocular movement, motor strength, ataxia, dysarthria, and sensory loss. A trained observer rates the patient's ability to answer questions and perform activities. Ratings for each item are scored with 3 to 5 grades with 0 as normal, and there is an allowance for untestable items. The level of stroke severity as measured by the NIH stroke scale scoring system: 0= no stroke, 1-4= minor stroke, 5-15= moderate stroke, 15-20= moderate/severe stroke, 21-42= severe stroke. In the present invention the term "higher score" refers to a score from 5 to 42 in the NIH stroke scale scoring system.

In another embodiment the methods of the invention further comprises a clinical predictor indicative of clinical outcome of a patient after suffering ischemic stroke.

The term "clinical predictor" refers to the best combination of medical sign, symptoms, and other findings in predicting the probability of a specific disease or outcome.

In a preferred embodiment the clinical predictor is Dragon Score and wherein if the DRAGON score is higher than 3, the clinical outcome is more negative or the patient is a preferred candidate for a therapy in a specialized stroke unit.

The term "Dragon score" refers to a tool to predict which stroke patients will have negative outcome, so they will need additional therapies. The DRAGON score had an accuracy of 84% in predicting three-month outcomes for ischemic stroke patients who received the tissue plasminogen activator (tPA) within four and a half hours of symptom onset, Strbian D. et al., Neurology. 2012 Feb 7;78(6):427-32., is based on scale of 0 to 10 points derived from several factors:
- (Hyper)Dense cerebral artery sign/early infarct signs on admission CT scan
- Prestroke modified Rankin scale
- Age
- Glucose levels at baseline
- Onset-to-treatment time
- Baseline National Institutes of Health stroke scale score

Other clinical predictors can be used in the present invention, such as Astral Score or Hotel Score.

Astral Score is a simple integer-based score to predict functional outcome using 6 readily available items at hospital admission: Age (A), severity of stroke (S) measured by admission NIH Stroke Scale score, stroke onset to admission time (T), range of visual fields (R), acute glucose (A), and level of consciousness (L) (Ntaios G. et al Neurology. 2012 Jun 12;78(24):1916-22).

Hotel Score is based on Hypotension, Oxygen saturation, low Temperature, ECG changes and Loss of independence, and quickly identifies patients at a low and high risk of death between 15 min and 24 h after admission, (Kellett J. et al Resuscitation. 2008 Ju1;78(1):52-8).

Other clinical predictor tools suitable for using in the present invention are G-score (Gompertz, Journal of Neurology, Neurosurgery, and Psychiatry 1994;57:932-935), Alberta Stroke Program Early CT score (ASPECTS) which is based on a systematic review of parenchymal changes observed on noncontrast CT (NCCT) scans, Boston Acute Stroke Imaging Scale (BASIS) (Torres-Mozqueda F. et al, AJNR Am J Neuroradiol. 2008 Jun;29(6):1111-7) or Glasgow Outcome Scale (GOS) (Miah T. et al., J Medicine 2009; 10 (supplement 1) : 11-14)

### Method for designing an individual therapy for a subject suffering ischemic stroke

In another aspect, the invention relates to an *in vitro* method for designing an individual therapy for a subject suffering ischemic stroke (second method of the invention) comprising
a) determining the level of a marker selected from gelsolin, CysA, DPYSL2 or a combination thereof, in a sample from said patient, and
b) comparing said level with a reference value for each marker,
wherein if the level of gelsolin or CysA in a sample from said patient is higher than a reference value for each marker and/or if the level of DPYSL2 in a sample from said patient is lower than the reference value, is indicative that the patient is a candidate for a therapy in a specialized stroke unit.

The terms "designing a therapy", as used herein, relates to the determination of a therapeutic intervention designed to cure a disease or palliate the symptoms associated with one or more diseases or conditions. In the particular case of a stroke therapy, it is understood any therapy which abolishes, retards or reduces the symptoms associated with stroke and, more in particular, with ischemic stroke.

As will be understood by those skilled in the art, the therapy, although preferred to be, need not be adequate for 100% of the subjects selected according to the second method of the invention. The term, however, requires that a statistically significant portion of subjects respond to the therapy. Whether the response to the therapy in a population of subjects is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.2, 0.1 or 0.05.

The terms "patient" and "ischemic stroke" have been described above.

In a first step, the method for designing an individual therapy for a subject suffering ischemic stroke according to the invention comprises determining the level of a marker selected from gelsolin, CysA, DPYSL2 or a combination thereof, in a sample from a patient.

The terms "gelsolin" "CysA" and "DPYSL2" have been defined in the context of the first method of the invention and are equally applicable to the second method.

In a preferred embodiment, the second method of the invention comprises determining the levels of one of the markers selected from CysA, gelsolin or DPYSL2. In another preferred embodiment, the methods of the invention comprise determining the levels of a combination of any two markers selected form the group consisting of CysA and gelsolin, CysA and DPYSL2, gelsolin and DPYSL2. In another preferred embodiment the invention comprises determining the levels of gelsolin, CysA and DPYSL2.

In a preferred embodiment, the sample wherein the biomarker or biomarkers are determined is a biofluid. In another embodiment, the biofluid is blood, plasma, serum, urine or cerebrospinal fluid.

In one embodiment, when the levels of gelsolin or CysA are determined, the determination is carried out in plasma. In another embodiment, when the levels of DPYSL2 are determined, the determination is carried out in serum.

In a preferred embodiment, the expression levels of gelsolin, CysA and DPYSL2 can be determined by measuring the levels of mRNA encoded by the corresponding genes. In another embodiment, the expression levels of gelsolin, CysA and DPYSL2 can be determined by measuring the levels of proteins encoded by said genes, and the levels of variants thereof. In one embodiment, the levels of markers are determined by an immunological technique. In yet another embodiment, the immunological technique is ELISA.

In a preferred embodiment the patient has been treated with a thrombolytic agent. The term "thrombolytic agent" is used as defined above. In a preferred embodiment, the thrombolyic agents is selected from the group consisting of reteplase (r-PA or Retavase), alteplase (t-PA or Activase), urokinase (Abbokinase), prourokinase, anisoylated purified streptokinase activator complex (APSAC), staphylokinase (Sak) and streptokinase. In a preferred embodiment the thrombolytic agent is plasminogen activator. In a more preferred embodiment the plasminogen activator is tissue plasminogen activator (t-PA). Suitable doses of t-PA should be given within the first 3 hours of the onset of symptoms. Recommended total dose: 0.9 mg/kg (maximum dose should not exceed 90 mg) infused over 60 minutes. Load with 0.09 mg/kg (10% of the 0.9 mg/kg dose) as an intravenous bolus over 1 minute, followed by 0.81 mg/kg (90% of the 0.9 mg/kg dose) as a continuous infusion over 60 minutes. Heparin should not be started for 24 hours or more after starting alteplase for stroke. Said t-PA is given intravenously and in some cases may be given directly into an artery and should be given right away after the first symptoms of stroke start.

In a second step of the method for designing an individual therapy for a subject suffering ischemic stroke according to the invention comprises comparing the level/s of the marker/s with a reference value for each marker. The "reference value" has been defined above in respect of the first method of the invention and is equally applicable to the second method of the invention. The reference value or reference level can be an absolute value; a relative value; a value that has an upper or a lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

According to the present invention, the level of a biomarker is increased when the level of said biomarker in a sample is higher than a reference value. The levels of a biomarker are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more higher than the reference value.

Likewise, in the context of the present invention, the level of a biomarker is decreased when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more lower than the reference value.

In a preferred embodiment, the reference value of gelsolin is about 19.87 ng protein/ml. In another embodiment, the reference value of CysA is about 31.76 ng protein/ml. In another embodiment, the reference value of DPYSL2 is about 2095.69 pg protein/ml.

In another embodiment the methods of the invention further comprises determining one or more clinical parameters. In a preferred embodiment the clinical parameter is selected from age, NIHSS score, presence of diabetes mellitus, previous stroke and sex, wherein old age, higher NIHSS score, suffering diabetes mellitus, previous stroke, and being men are indicative of that the patient is a candidate for a therapy in a specialized stroke unit.

In preferred embodiments, parameters which indicate that a patient is a candidate for a therapy in a specialized stroke unit include, without limitation, old age and preferably, age above 60, high NIHSS score, presence of diabetes mellitus, masculine sex and DRAGON score higher than 3.

Other clinical predictors can be used in the present invention, such as Astral Score, Hotel Score, G-score, Alberta Stroke Program Early CT score (ASPECTS), Boston Acute Stroke Imaging Scale (BASIS) or Glasgow Outcome Scale (GOS).

Once the levels of the different biomarkers in the sample of the patient have been compared with reference levels for each biomarker, a patient can be considered as a candidate for therapy in a specialized Stroke Unit.

The term "treatment" includes any process, action, application, therapy, or the like, wherein a subject (or patient), including a human being, is provided medical aid with the object of improving the subject's condition, directly or indirectly, or slowing the progression of a condition or disorder in the subject, or ameliorating at least one symptom of the disease or disorder under treatment.

There is no limitation as to the type of therapy that can be applied to the patients selected according to the method of the invention as long as it can be used in stroke treatment. The stroke therapeutic agent is preferably selected from the group comprising a reperfusion therapy, hypothermia, calcium channel blocker, glutamate, an NMDA receptor antagonist, an antioxidant, an apoptosis inhibitor, statins and minocycline-based antibiotics. Also the new cell therapies based on the neurorepair properties of neural stem cells, endothelial progenitor cells, etc and the growth factors released by those therapies. Alternatively, the therapy can be a therapy in a specialized stroke unit.

"Therapy in a specialized Stroke Unit" as used herein refers to therapies in areas inside of a hospital where physicians, nurses, and other ancillary personnel who have ample training and experience in stroke management, provide care for acute stroke patients. Management of acute stroke patients in this type of medical setting has been shown by several studies to improve a patient's chance of recovery and independent living. A systematic review of the randomized trials that have compared organized inpatient (stroke unit) care with contemporary conventional care has indicated that stroke patients who are managed in an organized (stroke unit) setting are less likely to die, remain physically dependent, or require long-term institutional care (Stroke Unit Trialists' Collaboration BMJ. 1997;314:1151-1159). Secondary complications of stroke (eg, chest infection, venous thromboembolism) could possibly be prevented through improved assessment procedures and early active rehabilitation.

Suitable therapies that can be applied to the patients selected according to the method of the invention include any therapy aimed at maximizing the individual functional abilities (rehabilitation), including speech therapy to relearn talking and swallowing, occupational therapy to regain as much function dexterity in the arms and hands as possible, and physical therapy to improve strength and walking.

Particularly, therapies to be applied in a specialized stroke unit are those included in the European Stroke Organization (ESO) Guidelines. By way of illustration those therapies and strategies include intermittent monitoring of neurological status, pulse, blood pressure, temperature and oxygen saturation is recommended for 72 hours in patients with significant persisting neurological deficits; oxygen should be administered if sPO2 falls below 95%, regular monitoring of fluid balance and electrolytes is recommended in patients with severe stroke or swallowing problems; normal saline (0.9%) is recommended for fluid replacement during the first 24 hours after stroke; routine blood pressure lowering is not recommended following acute stroke; abrupt blood pressure lowering should be avoided; low blood pressure secondary to hypovolaemia or associated with neurological deterioration in acute stroke should be treated with volume expanders; monitoring serum glucose levels is recommended; treatment of serum glucose levels >180mg/dl (>10mmol/l) with insulin titration is recommended; severe hypoglycaemia (<50 mg/dl [<2.8 mmol/l]) should be treated with intravenous dextrose or infusion of 10-20% glucose; the presence of pyrexia (temperature >37.5°C) should prompt a search for concurrent infection; treatment of pyrexia (>37.5°C) with paracetamol and fanning is recommended; specific stroke treatments such as thrombolytic therapy, early antithrombotic treatment or treatment of elevated intracranial pressure; prevention and management of complications such as infections after stroke should be treated with appropriate antibiotics; prophylactic administration of antibiotics is not recommended, and levofloxacin can be detrimental in acute stroke patients; early rehydration and graded compression stockings are recommended to reduce the incidence of venous thromboembolism; early mobilization is recommended to prevent complications such as aspiration pneumonia, DVT and pressure ulcers; low-dose s.c. heparin or low molecular weight heparins should be considered for patients at high risk of DVT or pulmonary embolism; administration of anticonvulsants is recommended to prevent recurrent seizures; Calcium/vitamin-D supplements are recommended in stroke patients at risk of falls; bisphosphonates (alendronate, etidronate and risedronate) are recommended in women with previous fractures; in stroke patients with urinary incontinence, specialist assessment and management is recommended; swallowing assessment is recommended but there are insufficient data to recommend a specific approach for treatment; oral dietary supplements are only recommended for non-dysphagic stroke patients who are malnourished; early commencement of nasogastric (NG) feeding (within 48 hours) is recommended in stroke patients with impaired swallowing.

Moreover, rehabilitation is also included in therapies to be applied in a specialized stroke unit. By way of illustration those therapies and strategies include rehabilitation should be continued after discharge during the first year after stroke; early initiation of rehabilitation is recommended; it is recommended that the duration and intensity of rehabilitation is increased; physiotherapy is recommended, but the optimal mode of delivery is unclear; occupational therapy is recommended; rehabilitation must be considered for all stroke patients, but there is limited evidence to guide appropriate treatment for the most severely disabled; drug therapy and non-drug interventions are recommended to improve mood, drug therapy should be considered to treat post stroke emotionalism; tricyclic or anticonvulsant therapy are recommended to treat post-stroke neuropathic pain in selected patients; botulinum toxin should be considered to treat post-stroke spasticity, but functional benefits are uncertain.

### Method for diagnosing a silent cerebrovascular disease

In another embodiment, the invention relates to an *in vitro* method for diagnosing a silent cerebrovascular disease (third method of the invention) comprising:
a) determining the level of a marker selected from gelsolin or DPYSL2 or a combination thereof, in a sample from said patient, and
b) comparing said level with a reference value for each marker,
wherein if the level of DPYSL2 in a sample from said patient is higher than a reference value for said marker or if the level of gelsolin in a sample from said patient is lower than a reference value for said marker is indicative that the patient has a silent cerebrovascular disease.

The term "Diagnosing/diagnosis", as used herein, means identifying the presence or nature of a silent cerebrovascular disease.

The term "cerebrovascular disease" as used herein, includes all disorders (1) in which an area of the brain is transiently or permanently affected by ischemia or bleeding and (2) one or more of the cerebral blood vessels are involved in the pathological process or a combination of (1) and (2). The pathological process includes abnormalities of the blood vessel wall, vessel stenosis or occlusion by thrombus or embolus, and altered permeability to plasma and blood cells.

These types of cerebrovascular diseases include lacunar and other strokes and minor hemorrhages.

These cerebrovascular diseases are termed "silent" because they typically affect "silent" regions of the brain that do not cause a noticeable change in an afflicted person's motor functions such as contralateral paralysis, slurred speech, pain, or an alteration in the sense of touch. A silent stroke typically affects regions of the brain associated with various thought processes, mood regulation and cognitive functions and is a leading cause of vascular cognitive impairment and may also lead to a loss of urinary bladder control. Thus, the term "silent cerebrovascular disease", as used herein, refers to a cerebrovascular disease that does not have any outward symptoms, and the patient is typically unaware they have suffered a cerebrovascular disease. Despite not causing identifiable symptoms a silent cerebrovascular disease still causes damage to the brain, and places the patient at increased risk for both transient ischemic attack and major cerebrovascular disease in the future.

The particulars of "gelsolin", "DPYSL2", "higher level", "lower level" and "reference value" have been previously described in connection with the first and second method of the invention, whose particulars are hereby incorporated. In a particular embodiment of the method for diagnosing a silent cerebrovascular disease, the reference value refers to the level of the biomarkers in a sample of a healthy subj ect, particularly subjects without any cerebral event.

In a preferred embodiment, the third method of the invention comprises determining the levels of one of the markers selected from gelsolin or DPYSL2. In another preferred embodiment, the methods of the invention comprise determining the levels of a combination of markers gelsolin and DPYSL2.

In a preferred embodiment, the expression levels of gelsolin and DPYSL2 can be determined by measuring the levels of mRNA encoded by the corresponding genes. In another embodiment, the expression levels of gelsolin and DPYSL2 can be determined by measuring the levels of proteins encoded by said genes, and the levels of variants thereof. In one embodiment, the levels of markers are determined by an immunological technique. In yet another embodiment, the immunological technique is ELISA.

In a preferred embodiment, the sample wherein the biomarker or biomarkers are determined according to the third method of the invention is a biofluid. In another embodiment, the biofluid is blood, plasma, serum, urine or cerebrospinal fluid.

In one embodiment, when the levels of gelsolin are determined, the determination is carried out in plasma. In another embodiment, when the levels of DPYSL2 are determined, the determination is carried out in serum.

According to the present invention, the level of a biomarker is increased when the level of said biomarker in a sample is higher than a reference value. The levels of a biomarker are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more higher than the reference value.

Likewise, in the context of the present invention, the level of a biomarker is decreased when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more lower than the reference value.

In another preferred embodiment of the third method of the invention, the patient is selected from a healthy subject or a subject having vascular risk factors.

The term "healthy subject" relates to a subject free of diseases, particularly free of cerebrovascular diseases.

The term "subject having vascular risk factors" refers to a subject having a risk factor selected from high blood pressure and atrial fibrillation, high blood cholesterol levels, diabetes, cigarette smoking (active or passive), heavy alcohol consumption and drug use, lack of physical activity, obesity, processed red meat consumption and unhealthy diet, eldery, being woman or having African or Asian ancestry.

### Kit of the invention

In another embodiment, the invention relates to a kit comprising reagents for detecting the level of a marker selected from gelsolin, CysA, DPYSL2 or a combination thereof.

The term "kit", as used herein, refers to a product containing the different reagents necessary for carrying out the methods of the invention packed so as to allow their transport and storage. Materials suitable for packing the components of the kit include crystal, plastic (e.g. polyethylene, polypropylene, polycarbonate), bottles, vials, paper, or envelopes.

In a preferred embodiment, the kit comprises reagents for determining the levels of a combination of any two markers selected form the group consisting of gelsolin, CysA and DPYSL2, including CysA and gelsolin, CysA and DPYSL2 and gelsolin and DPYSL2. In another preferred embodiment the invention comprises determining the levels of gelsolin, CysA and DPYSL2.

Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instructions.

The reagents of the kit include compounds that bind specifically to the marker proteins. Preferably, said compounds are antibodies, aptamers or fragments thereof. More preferably, said compounds are antibodies or fragments thereof.

The antibodies of the kit of the invention can be used according to techniques known in art for determining the protein expression levels, such as, for example, flow cytometry, Western blot, ELISA, RIA, competitive EIA, DAS-ELISA, techniques based on the use of biochips, protein microarrays, or assays of colloidal precipitation in reactive strips.

The antibodies can be fixed to a solid support such as a membrane, a plastic or a glass, optionally treated to facilitate the fixation of said antibodies to the support. Said solid support comprises, at least, a set of antibodies which specifically recognize the marker, and which can be used for detecting the levels of expression of said marker.

Additionally, the kits of the invention comprise reagents for detecting a protein encoded by a constitutive gene. The availability of said additional reagents allows normalizing the measurements performed in different samples (for example, the sample to be analyzed and the control sample) to rule out that the differences in the expression of the biomarkers are due to a different quantity of total protein amount in the sample more than the real differences in the relative levels of expression. The constitutive genes in the present invention are genes that are always active or being transcribed constantly and which encode for proteins that are expressed constitutively and carry out essential cellular functions. Proteins that are expressed constitutively and can be used in the present invention include, without limitation, β-2-microglobulin (B2M), ubiquitin , 18-S ribosomal protein, cyclophilin, GAPDH, PSMB4, tubulin and actin.

In a preferred embodiment, the reagents for assaying the levels of the different biomarkers comprise at least 10%, at least 20%, at least 30%, at least 40%, at least 50%, at least 60%, at least 70%, at least 80%, at least 90% or at least 100% of the total amount of reagents for assaying biomarkers forming the kit. Thus, in the particular case of kits comprising reagents for assaying the levels of gelsolin, CysA and/or DPYSL2, the reagents specific for said biomarkers (i.e. antibodies which bind specifically to gelsolin, CysA and DPYSL2) comprise at least 10%, at least 20%, at least 30%, at least 40%, at least 50%, at least 60%, at least 70%, at least 80%, at least 90% or at least 100% of the antibodies present in the kit.

In another aspect, the invention refers to the use of a kit of the invention for determining the clinical outcome of a patient suffering ischemic stroke, for designing an individual therapy for a subject suffering ischemic stroke or for diagnosing a silent cerebrovascular disease.

The invention will be described by way of the following examples which are to be considered as merely illustrative and not limitative of the scope of the invention.

### EXAMPLES

### MATERIAL AND METHODS

### Brain tissue samples

Brain samples preparation and demographic data was performed as previously described in Cuadrado E. et al, (Exp. Neurol., 2010, 69(11), 1105-15). Briefly, tissue samples from ischemic ipsilateral (infarct core and peri-infarct area) and healthy contralateral hemispheres of 3 ischemic stroke patients were obtained within the first 6 h after death. Cortical brain areas from 3 patients who died because of non-inflammatory and non-neurological disease were used as controls. All samples were snap frozen in liquid nitrogen and immediately stored at -80°C until homogenization.

### Blood samples

Consecutive patients with an acute ischemic stroke admitted to the emergency department of the Vall d'Hebron Hospital (Barcelona, Spain) within the first 4.5 h after symptoms onset, were recruited from February 2007 to September 2009. Stroke onset was defined as the last time the patient was known to be asymptomatic. All patients received thrombolytic treatment.

The first part of the study is retrospective and includes 60 patients who underwent a standardized protocol of clinical and neuroradiological assessments as previously described by Mendioroz M, et al., (Neurol., 2011, 258(3), 486-93). Stroke severity was assessed by using the National Institutes of Health Stroke Scale (NIHSS) (Brott T, J. Med., 2000, 343, 710-22). We defined neurological improvement as a decrease in NIHSS score by ≥ 4 points and neurological worsening as an increase in score by ≥ 4 points at 24 h, 48 h and discharge (Brott TG, Stroke, 1992, 23, 632-40). Functional outcome was evaluated at third month after the event by means of the modified Rankin Scale (mRS); disability of patients was considered when mRS score is higher than 2. In-hospital death by any cause was also considered.

The second part of the study is prospective and includes 36 subjects from a cohort of essential hypertensive individuals between 50 to 70 years of age (ISSYS study: Investigating Silent Stroke in hYpertensives - a magnetic resonance imaging Study). All participants were examined by 1.5 T magnetic resonance imaging (MRI). Brain infarcts were defined as lesions ≥ 3 mm with signal characteristics of CSF in all pulse sequences and with a hyperintense rim in FLAIR sequence (Riba, I, J Neurol Sci, 2012, Jul 24, Epub ahead of print).

Peripheral blood samples were drawn from stroke patients at admission before any treatment was given. In a few cases, blood was also drawn 1 h and 24 h after thrombolytic treatment and at discharge of stroke patients (temporal profile). Blood extracted from 5 healthy volunteer controls was used to set reference values. Regarding ISSYS individuals, blood was drawn at the moment of the inclusion visit. Plasma or serum were immediately separated by centrifugation at 3,000 rpm for 15 min at 4°C and stored at -80°C until use.

### Ethics statement

The local ethical committees approved both studies (i.e., human brain tissue and blood sampling), and written consent was obtained from all subjects or relatives in accordance with the Helsinki declaration.

### Proteomics analysis

### 1- Two-dimensional fluorescence Difference Gel Electrophoresis (2D-DIGE)

Sample preparation for 2D-DIGE was performed as published in Cuadrado E et al, (Exp. Neurol., 2010, 69(11), 1105-15). Briefly, brain tissue homogenates from each area were alternatively stained with Cy3 or Cy5 cyanine dyes to avoid possible bias by labeling efficiency; internal standard pooled sample was labeled with Cy2 dye and was used for quantitative comparisons (Alban A. et al., Proteomics, 2003, 3; 36-443). After separation by 2-dimensional gel electrophoresis, fluorescence images of each gel were acquired and image analysis and statistical quantification of relative protein abundance were performed (Cuadrado E et al, Exp. Neurol., 2010, 69(11), 1105-15).

### 2- Protein identification by MALDI-TOF/TOF MS

Protein spots were excised from the gels using an automated Spot Picker (GE Healthcare). In-gel digestion was performed as previously described (Shevchenko A. et al., Anal. Chem., 1996, 68, 850-58) using autolysis stabilized trypsin (Promega, Fitchburg, WI). Tryptic digests were purified using ZipTip microtiter plates (Millipore, Cork, Ireland). Identification of the proteins was carried out as described in Cuadrado E. et al, (Exp. Neurol., 2010, 69(11), 1105-15). Briefly, matrix-assisted laser desorption/ionization mass spectrometry (MALDI MS) analysis of tryptic peptides was performed on an Ultraflex TOF-TOF Instrument (Bruker, Bremen, Germany). Filtration and normalization of the spectra generated a final peak list used for identification of the proteins by the peptide mass fingerprinting method. Mascot algorithm was used to search the MSDB database for human proteins. For the identified fragments of proteins, searched protein mass was set to the observed mass according to the gel position. Criterion for positive identification was a significant Mascot probability score (score > 64; p < 0.05).

### 3- Protein identification by ESI LTQ-OT MS

Missing protein spots that were not identified by MALDI-TOF/TOF were processed by an electrospray ionization hybrid mass spectrometry (ESI MS) on a linear trap quadrupole-orbitrap LTQ-OT Velos (Thermo Electron, San Jose, CA, USA) equipped with a NanoAcquity system from Waters. Peptides were trapped on a home-made 5 µm 200 Å Magic C18 AQ (Michrom) 0.1 × 20 mm pre-column and separated on a home-made 5 µm 100 Å Magic C18 AQ (Michrom) 0.75 × 150 mm column with a gravity-pulled emitter. The analytical separation was run for 65 min using a gradient of H2O/FA 99.9%/0.1% (solvent A) and CH3CN/FA 99.9%/0.1% (solvent B). The gradient was run as follows: 0-1 min 95% A and 5% B, then to 65% A and 35% B at 55 min, and 20% A and 80% B at 65 min at a flow rate of 220 nL/min. For MS survey scans, the OT resolution was set to 60,000 and the ion population was set to 5 × 105 with an m/z window from 400 to 2,000. Five precursor ions were selected for collision-induced dissociation (CID) in the LTQ. For this, the ion population was set to 1 × 104 [7e3 for velos] (isolation width of 2 m/z). The normalized collision energies were set to 35% for CID.

Protein identification peak lists were generated from raw data using the embedded software from the instrument vendor (extract_MSN.exe). The monoisotopic masses of the selected precursor ions were corrected using an in-house written Perl script (Scherl A. et al., Proteomics, 2008, 8 (14), 2791-7). The corrected mgf files were searched against UniProtSP database (v56.9 of 03-Mar-2009) using Phenyx (GeneBio, Geneva, Switzerland). Homo Sapiens taxonomy was specified for database searching. The parent ion tolerance was set to 10 ppm. Variable amino acid modifications were oxidized methionine. Trypsin was selected as the enzyme, with one potential missed cleavage, and the normal cleavage mode was used. Only one search round was used with selection of "turbo" scoring. The peptide p value was 1 E-2 for LTQ-OT data. False-positive ratios were estimated using a reverse decoy database (Elias JE. et al., Nat. Methods, 2007, 4(3), 207-14). All datasets where searched once in the forward and once in the reverse database. Separate searches were used to keep the database size constant. Protein and peptide score were then set up to maintain the false positive peptide ratio below 1%. This resulted in a slight overestimation of the false-positive ratio (Elias JE, Nat. Methods, 2007, 4(3), 207-14). For all analyses, only proteins matching at least two different peptide sequences were kept.

### Immunoassays

Replication study of the selected candidates was performed by enzyme-linked immunosorbent assay (ELISA) commercial kits in blood samples, either plasma or serum. Brain glycogen phosphorylase (GPBB), dihydropyrimidinase-related protein 2(DPYSL2) and gelsolin ELISAs were from Cusabio Biotech Co. (Newark, DE, USA). Rho GDP-dissociation inhibitor-alpha (Rho GDI-1), septin-5, calcyclin-binding protein (CACYBP), contactin-1 (CNTN-1) and cystatin A (CysA) ELISA kits were purchased to USCN Life Science Inc. (Wuhan, P.R. China). All ELISAs were performed according to the manufacturer's instructions by trained technicians blinded to clinical details.

Each sample was assayed twice and the mean value of both measurements was used. All samples which intra-assay coefficient of variation (CV) in concentration was higher than 20% were excluded from the analysis. The mean inter-assay CV was lower than 30% for all proteins assessed.

### Statistical analysis

Whole analysis was performed with SPSS 15.0 software, unless contrary is stated. Those variables normally distributed (Kolmogorov-Smirnov test with p>0.05) were analyzed by Student's t test or ANOVA; mean and standard deviation (SD) values are given. For variables with non-normal distribution Mann-Whitney U or Kruskal-Wallis test were used; median and interquartile range are reported. Differences among categorical variables were assessed by Pearson chi-squared test. Paired test analysis was performed to assess differences among biomarker levels in temporal profiles.

A Classification Tree (CRT; Gini method) analysis was used to find cut-off points for each individual biomarker. The minimum number of patients used for the nodes were established at 15 for parent nodes and 5 for terminal nodes.

Forward stepwise multivariate logistic regression models for each end-point (disability at 3rd month, in-hospital mortality) were constructed with all clinical variables associated with end-point at p<0.1 (Table 3). Odds ratio (OR) and 95% confidence interval (CI) were adjusted by clinical variables known to be related to stroke prognosis (i.e. age, NIHSS at admission and sex). Baseline levels of biomarkers using cut-off points or the combination of biomarkers with their cut-off points were added to the correspondent clinical model to find the new model.

The areas under the ROC curve (AUC) from models with biomarker or combination of biomarkers were compared with AUC from clinical model by using MedCalc software. Using R software (Hmisc and PredictABEL packages), net reclassification improvement (NRI) and integrated discrimination improvement (IDI) indexes were calculated to assess the added value of the biomarker or combination of biomarkers to the clinical models to predict outcome (Pencina MJ, Stat. Med., 2008, 27, 157-72; Pickering JW. et al, Clin J Am Soc Nephrol. 2012 Jun 7). In the case of NRI test, pre-specified clinically relevant thresholds of predicted risk (<10% and >90%) were used to calculate reclassification of patients.
In all cases a p<0.05 was considered significant at a 95% confidence level.

### Example 1- Proteins showing differential expression profile

Brain homogenates obtained from the contralateral hemisphere (CL), peri-infarct (PI) and infarct core (IC) from 3 stroke patients and 3 controls (CON) were used to create a 2-dimensional protein map. On average, 1,442 ± 231 protein spots were found in the gels and 132 spots have at least 1.5-fold changes in their relative expression between the different brain tissue areas. 42 spots and 39 unique proteins were successfully identified by MALDI-TOF/TOF MS and MASCOT algorithm (Cuadrado E. et al, J. Neuropathol. Exp. Neurol., 2010, 69(11), 1105-15).

90 missing protein spots were processed by ESI LTQ-OT MS, with higher accuracy and resolution, and 20 spots and 12 new unique proteins were identified (Table 1). From these, 4 proteins were over-expressed and 8 decreased in IC.

**Table 1. Proteins identified from 2D-DIGE (pH 3-10) of human brain homogenates by ESI LTQ-OT MS.**

| **Spot No.** | **Protein name** | **Accession No.** | **MW** | **pI** | **Mascot Score** | **Peptides** |
|---|---|---|---|---|---|---|
| Blood coagulation 1074 | Fibrinogen gamma chain (FGG) | P02679 | 52,106 | 5.37 | 716 | 23 |
| Carbohydrate metabolism 1272 | Citrate synthase (CS), mitochondrial | 075390 | 51,908 | 8.45 | 567 | 19 |
| Cell adhesion molecules 104 | Contactin-1 (CNTN-1) | Q12860 | 114,104 | 5.62 | 548 | 16 |
| Cytoskeleton 292 | Gelsolin | P06396 | 86,043 | 5.9 | 284 | 8 |
| Glycolysis 1668 | L-lactate dehydrogenase A chain (LDH-A) | P00338 | 36,950 | 8.44 | 181 | 8 |
| Immunity 1027 | Ig gamma chain C region (IGHG1) | P01857 | 36,596 | 8.46 | 455 | 11 |
| Oxidoreductase 2162 | Alcohol dehydrogenase (ADH) | P00325 | 36,892 | 6.32 | 433 | 16 |
| 1023 | Alpha-aminoadipic semialdehyde dehydrogenase (aAASA-DH) | P49419 | 55,845 | 6,44 | 461 | 11 |
| Proteinase inhibitor 1746 | Cystatin A (CysA) | P01040 | 11,000 | 5.38 | 284 | 7 |
| Transferase | | | | | | |
| 1957 | Glutathione S-transferase Mu2 (GSTM2) | P28161 | 25,899 | 6 | 240 | 10 |
| 1441 | Phosphoserine aminotransferase (PSAT) | Q9Y617 | 40,796 | 7.56 | 180 | 4 |
| Ubiquitination 1844 | Calcyclin-binding protein (CACYBP) | Q9HB71 | 26,308 | 8.28 | 267 | 15 |

| | | | | | | |
|---|---|---|---|---|---|---|
| **Spot No** Protein spot numbers assigned in DeCyder analysis and corresponded to the 2D electrophoresis gels (*Cuadrado E, 2010*) Proteins are listed and grouped according to functional classification **Accesion No** Accession number in Uni-Prot/Swiss-Prot **MW** Theoretical molecular weight, Da **pI** Theoretical isoelectrical point **Peptides** Number of peptide masses matching to the identified protein | | | | | | |

It was not possible to obtain MS sequence data to identify the remaining 70 protein spots. Thus, in total 51 proteins were recognized as differently expressed depending on the brain area studied. The detailed workflow is given in Figure 1.

### Example 2- Verification of potential candidate biomarkers with ELISAs

To identify potential biomarkers from the 51 proteins identified by MS studies, 8 candidates were selected from different functional categories based on the availability of commercial immunoassays. From the 39 proteins identified by MALDI-TOF/TOF MS four proteins were chosen as candidates, two of them having less expression in IC (GPBB and Septin-5) and DPYSL2 and Rho GDI-1 being higher in IC (Cuadrado E, 2010). On the other hand, CACYBP and gelsolin (lower expression in IC) and CNTN-1 and CysA (higher expressed in IC) (Table 1) were selected from the 12 proteins identified by ESI LTQ-OT MS.

When assayed in blood samples either GPBB, Septin-5 and CACYBP were non-detectable neither in plasma nor in serum. Finally, ELISAs were performed for gelsolin and CysA (plasma samples) and Rho IDI-1, DPYSL2 and CNTN-1 (serum samples) in 60 ischemic stroke patients followed-up till 3rd month after stroke. The median age was 77 years (interquartile range [IQR], 65.5 - 81), 50% were men and the NIHSS score at admission was 14.7 ± 5.9. Thirteen percent of patients worsened and 58.7% improved within 48 h. Furthermore, we have explored factors that might influence on the blood levels of our biomarker candidates finding that Rho GDI-1 and DPYSL2 were elevated and gelsolin was reduced in those patients that suffered a previous stroke (p = 0.002, p = 0.004 and p = 0.021, respectively) and CysA was elevated in hypertensive patients (p = 0.032) (Table 2). No significant relationship was found between our biomarkers levels and stroke severity neither at 24 h, 48 h (Table 2) nor at discharge moment.

**Table 2. Biomarkers blood levels regarding demographic, clinical and risk factors.**

| **Factors** | **Gelsolin (ng/mL)** | | | **Cystatin-A (ng/mL)** | | | **Rho GDI-1 (ng/mL)** | | |
|---|---|---|---|---|---|---|---|---|---|
| | **Yes** | **No** | **p-value,** | **Yes** | **No** | **p-value** | **Yes** | **No** | **p-value** |
| Male | 20.0±5 | 19.8±4 | 0.842 | 24.8±6 | 26.6±9 | 0.384 | 18.3±15 | 16.4±12 | 0.602 |
| Smoker | 21.1±5 | 19.9±4 | 0.512 | 24.7±5 | 25.9±8 | 0.722 | 15.2±10 | 17.7±14 | 0.680 |
| Hypertension | 19.6±4 | 20.7±3 | 0.376 | 27.0±8 | 22.3±5 | **0.032** | 17.5±13 | 17.3±15 | 0.971 |
| Diabetes mellitus | 18.9±3 | 20.3±5 | 0.267 | 27.3±8 | 25.0±7 | 0.295 | 19.1±16 | 16.9±13 | 0.619 |
| Dyslipidemia | 19.4±4 | 20.4±4 | 0.384 | 24.6±8 | 26.4±7 | 0.366 | 16.0±12 | 18.2±15 | 0.565 |
| Atrial fibrillation | 20.6±5 | 19.7±4 | 0.473 | 26.8±6 | 25.1±8 | 0.438 | 14.4±11 | 18.8±15 | 0.281 |
| Heart disease | 19.1±5 | 20.2±4 | 0.386 | 27.8±9 | 25.0±7 | 0.235 | 23.5±15 | 15.7±13 | 0.078 |
| Previous stroke | 16.6±4 | 20.4±4 | **0.021** | 28.0±11 | 25.2±7 | 0.314 | 30.6±14 | 15.2±12 | **0.002** |
| Atherothrombotic | 19.2±3 | 20.1±4 | 0.512 | 28.0±10 | 24.9±7 | 0.183 | 20.4±16 | 16.6±13 | 0.398 |
| Cardioembolic | 20.0±5 | 19.9±4 | 0.913 | 25.4±6 | 25.8±9 | 0.846 | 14.0±10 | 20.3±15 | 0.078 |
| Hemorrhagic transformation | 19.0±4 | 20.2±4 | 0.365 | 27.1±9 | 25.2±7 | 0.434 | 22.4±17 | 16.0±12 | 0.154 |
| SICH | 19.7±1 | 20.0±4 | 0.922 | 24.0±13 | 25.7±7 | 0.776 | 27.2±29 | 17.0±13 | 0.302 |
| Neurological improvement 48 h | 19.3±4 | 20.2±4 | 0.482 | 25.8±7 | 24.9±9 | 0.699 | 19.5±14 | 19.0±15 | 0.917 |
| Neurological worsening 48 h | 18.6±3 | 19.9±4 | 0.486 | 29.0±14 | 24.8±7 | 0.448 | 14.5±16 | 20.1±14 | 0.392 |
| 3^{rd} month mRS > 2 | 20.7±4 | 18.2±3 | **0.042** | 26.9±8 | 22.6±5 | 0.052 | 15.8±13 | 21.5±13 | 0.159 |
| In-hospital death | 18.4±5 | 20.2±4 | 0.308 | 32.0±12 | 24.6±6 | 0.112 | 19.0±17 | 17.1±13 | 0.727 |

| **Factors** | **DPYSL2 (ng/mL) CNTN-1 (ng/mL)** | | | | | | | | |
|---|---|---|---|---|---|---|---|---|---|
| | **Yes** | **No** | **p-value** | **Yes** | **No** | **p-value** | | | |
| Male | 0.70 (0.38-1.54) | 0.56 (0.33-0.78) | 0.232 | 37.9±17 | 35.8±14 | 0.631 | | | |
| Smoker | 0.43 (0.36-0.67) | 0.65 (0.34-1.43) | 0.542 | 39.1±16 | 36.4±16 | 0.714 | | | |
| Hypertension | 0.66 (0.37-0.90) | 0.48 (0.34-2.05) | 0.598 | 37.7±17 | 34.7±10 | 0.461 | | | |
| Diabetes mellitus | 0.64 (0.39-0.78) | 0.66 (0.34-1.43) | 0.826 | 34.0±11 | 38.0±17 | 0.321 | | | |
| Dyslipidemia | 0.49 (0.33-0.88) | 0.66 (0.41-1.57) | 0.242 | 35.3±16 | 38.0±15 | 0.550 | | | |
| Atrial fibrillation | 0.60 (0.28-0.74) | 0.69 (0.36-1.54) | 0.205 | 41.1±17 | 35.0±14 | 0.202 | | | |
| Heart disease | 0.80 (0.38-2.10) | 0.64 (0.33-0.90) | 0.160 | 37.9±21 | 36.5±13 | 0.788 | | | |
| Previous stroke | 1.85 (1.48-1.98) | 0.55 (0.33-0.90) | **0.004** | 51.1±23 | 34.9±13 | 0.142 | | | |
| Atherothrombotic | 0.78 (0.45-1.33) | 0.64 (0.32-1.15) | 0.353 | 32.6±14 | 38.2±16 | 0.283 | | | |
| Cardioembolic | 0.52 (0.28-0.78) | 0.78 (0.39-1.76) | 0.057 | 41.0±18 | 33.5±12 | 0.106 | | | |
| Hemorrhagic transformation | 0.65 (0.34-1.96) | 0.65 (0.35-0.91) | 0.552 | 39.4±19 | 36.1±15 | 0.533 | | | |
| SICH | 1.15 (0.32-1.98) | 0.65 (0.35-0.92) | 0.877 | 32.4±5 | z16 | 0.681 | | | |
| Neurological improvement 48 h | 0.48 (0.35-1.89) | 0.70 (0.34-1.15) | 0.813 | 33.4±15 | 37.5±17 | 0.428 | | | |
| Neurological worsening 48 h | 0.34 (0.32-0.86) | 0.68 (0.38-1.73) | 0.215 | 39.6±13 | z16 | 0.454 | | | |
| 3^{rd} month mRS > 2 | 0.54 (0.33-0.86) | 0.90 (0.43-2.21) | **0.045** | 36.2±15 | z16 | 0.604 | | | |
| In-hospital death | 0.88 (0.49-1.33) | 0.64 (0.33-0.92) | 0.589 | 44.2±18 | 35.6±15 | 0.176 | | | |

| | | | | | | | | | |
|---|---|---|---|---|---|---|---|---|---|
| Gelsolin, CysA, Rho GDI-1 and CNTN-1 are expressed as mean ± SD; DPYSL2 is expressed as median (lower-upper quartile). Statistically significant differences between groups are expressed as bold p-value. * statistical trend. SICH, symptomatic intracranial hemorrhage; mRS, modified Rankin scale. | | | | | | | | | |

### Example 3-Blood temporal profile

CysA showed differences among temporal profile, decreasing its level 1h after thrombolytic treatment administration (p<0.001) and recovering control reference level at discharge (Figure 2).

### Example 4-Long-term outcome

Patients functionally disabled at third month after stroke (N = 43, 71.7%) were older (p<0.001), had higher mRS score previous to stroke and higher NIHSS score at admission (p=0.063 and p=0.006, respectively) and had more diabetes mellitus (p=0.024) (Table 3). Regarding biomarkers, disabled patients had higher baseline plasma concentration of gelsolin (p = 0.042) and CysA (p = 0.052) and lower baseline serum concentration of DPYSL2 (p = 0.073) than those patients with a better long-term prognosis (Figure 3, Table 2). By means of individual CRT analysis we found these cut-off points associated to disability at 3rd month: gelsolin 19.87 ng/mL (71.8% sensitivity, 76.5% specificity), CysA 31.76 ng/mL (26.8% se., 100% sp.) and DPYSL2 2095.69 pg/mL (97.1 % se., 28.6% sp.) (Table 3).

**Table 3. Clinical characteristics and factors associated to long-term outcome by mRS at third month after stroke and in-hospital mortality (Univariate analysis).**

| **Factors** | **mRS < 2 (N = 17)** | **mRS > 2 (N = 43)** | **p-value** | **Survivor (N = 52)** | **Exitus (N = 8)** | **p-value** |
|---|---|---|---|---|---|---|
| Age median (IQR) | 64 (61-71) | 78 (75-82) | **0.0002** | 76 5(63-81) | 77 5 (77-82) | 0231 |
| NIHSS at admission mean ± SD | 11 4 ± 5 2 | 16 0 ± 5 7 | **0.006** | 13 8 ± 5 4 | 20 5 ± 5 8 | **0.002** |
| Sex (Male) % (n) | 52 9 (9) | 48 8(21) | 0774 | 50 (26) | 50 (4) | 1 000 |
| Smokers % (n) | 17 6(3) | 7 1 (3) | 0341 | 11 8 (6) | 0(0) | 0583 |
| Arterial hypertension % (n) | 58 8(10) | 76 7 (33) | 0209 | 71 2(37) | 75 (6) | 1 000 |
| Diabetes mellitus %(n) | 5 9 (1) | 37 2(16) | **0.024** | 30 8 (16) | 12 5 (1) | 0420 |
| Dyslipidemia % (n) | 41 2(7) | 39 5(17) | 0907 | 42 3 (22) | 25 (2) | 0457 |
| Atrial fibrillation % (n) | 17 6(3) | 37 2(16) | 0142 | 30 8 (16) | 37 5 (3) | 0699 |
| Heart disease % (n) | 23 5 (4) | 23 3 (10) | 1 000 | 25 (13) | 12 5 (1) | 0667 |
| Previous stroke % (n) | 5 9 (1) | 16 3 (7) | 0420 | 9 6 (5) | 37 5 (3) | 0 065 * |
| Vessel localization | | | 0638 | | | **0.025** |
| - MCA % (n) | 100 (17) | 90 7 (39) | | 96 2 (50) | 75 (6) | |
| TOAST | | | 0365 | | | 0391 |
| - Atherothrombotic % (n) | 17 6(3) | 23 3 (10) | | 21 2(11) | 25 (2) | |
| - Cardioembolic % (n) | 35 3 (6) | 48 8(21) | | 42 3 (22) | 62 5 (5) | |
| - Undetermined % (n) | 47 1 (8) | 27 9 (12) | | 36 5(19) | 12 5 (1) | |
| HT % (n) | 23 5 (4) | 23 3 (10) | 1000 | 23 1 (12) | 25 (2) | 1 000 |
| - Symptomatic HT % (n) | 0(0) | 4 7 (2) | 1 000 | 3 8 (2) | 0(0) | 1 000 |
| Gelsolin > 19 87 ng/mL % (n) | 23 5 (4) | 71 8 (28) | **0.001** | - | - | - |
| CysA > 24 63 ng/mL % (n) | 29 4 (5) | 61 (25) | **0.029** | - | - | - |
| Rho GDI-1 < 14 29 ng/mL % (n) | 68 8 (11) | 38 5(15) | **0.041** | - | - | - |
| DPYSL2 < 1 54 ng/mL % (n) | 42 9 (6) | 9 1 (3) | **0.013** | - | - | - |
| CysA > 34 35 ng/mL % (n) | - | - | - | 4 (2) | 62 5 (5) | **0.0002** |

| | | | | | | |
|---|---|---|---|---|---|---|
| mRS, modified Rankm scale, IQR, interquartile range, SD, standard deviation, TOAST, etiology stroke subtype classification, NIHSS, National Institutes of Health Stroke Scale Statistically significant differences between groups are expressed as bold p-value * trend included in multivariate analysis | | | | | | |

Multivariate logistic regression was performed and significant variables adjusted by NIHSS and sex, confirming diabetes mellitus (ORadj 9.3, p=0.051) and age (ORadj 1.1, p=0.009) as independent predictors for disability at 3rd month. When baseline gelsolin > 19.87 ng/mL or DPYSL2 < 2095.69 pg/mL were added to the clinical model, biomarkers were independent predictors of bad outcome. Moreover, several combinations of the three candidate biomarkers and their inclusion in models together with clinical data (i.e. age and diabetes mellitus or DRAGON score) were also independent predictors (Table 4).

| **Independent predictor disability at 3^{rd} month** | **Only clinical model** | | | **Only DRAGON score** | | |
|---|---|---|---|---|---|---|
| | **OR**_{adj} | **95% CI** | **p-value** | **OR** | **95% CI** | **p-value** |
| Age | 1.1 | 1.0-1.2 | 0.009 | - | - | - |
| Diabetes mellitus | 9.3 | 1.0-88 | 0.051 | - | - | - |
| DRAGON score | - | - | - | 2.4 | 1.3-4.3 | 0.004 |
| Gelsolin | - | - | - | - | - | - |
| DPYSL2 | - | - | - | - | - | - |
| CysA | - | - | - | - | - | - |
| Gelsolin + DPYSL2 | - | - | - | - | - | - |
| Gelsolin + CysA | - | - | - | - | - | - |
| DPYSL2 + CysA | - | - | - | - | - | - |
| Gelsolin + DPYSL2 + CysA | - | - | - | - | - | - |

| **Independent predictor disability at 3^{rd} month** | **Clinical + Gelsolin** | | | **DRAGON + Gelsolin** | | |
|---|---|---|---|---|---|---|
| | **OR_{adj}** | **95% CI** | **P-value** | **OR** | **95% CI** | **p-value** |
| Age | 1.1 | 1.0-1.2 | 0.019 | - | - | - |
| Diabetes mellitus | 14.8 | 1.2-186 | 0.036 | - | - | - |
| DRAGON score | - | - | - | 2.1 | 1.1-4.0 | 0.023 |
| Gelsolin | 8.9 | 1.7-48 | 0.010 | 6.4 | 1.3-30.8 | 0.021 |
| DPYSL2 | - | - | - | - | - | - |
| CysA | - | - | - | - | - | - |
| Gelsolin + DPYSL2 | - | - | - | - | - | - |
| Gelsolin + CysA | - | - | - | - | - | - |
| DPYSL2 + CysA | - | - | - | - | - | - |
| Gelsolin + DPYSL2 + CysA | - | - | - | - | - | - |

| **Independent predictor disability at 3rd month** | **Clinical + DPYSL2** | | | **DRAGON + DPYSL2** | | |
|---|---|---|---|---|---|---|
| | **OR_{adj}** | **95% CI** | **p-value** | **OR** | **95% CI** | **p-value** |
| Age | 1.2 | 1.1-1.4 | 0.005 | - | - | - |
| Diabetes mellitus | 51.5 | 0.8-3470 | 0.066 | - | - | - |
| DRAGON score | - | - | - | 2.5 | 1.3-5.0 | 0.008 |
| Gelsolin | - | - | - | - | - | - |
| DPYSL2 | 0.02 | 0.0-0.6 | 0.023 | 0.05 | 0.0-0.6 | 0.022 |
| CysA | - | - | - | - | - | - |
| Gelsolin + DPYSL2 | - | - | - | - | - | - |
| Gelsolin + CysA | - | - | - | - | - | - |
| DPYSL2 + CysA | - | - | - | - | - | - |
| Gelsolin + DPYSL2 + CysA | - | - | - | - | - | - |

| **Independent predictor disability at 3^{rd} month** | **Gelsolin + DPYSL2** | | | **Clinical + Gelsolin + DPYSL2** | | |
|---|---|---|---|---|---|---|
| | **OR** | **95% CI** | **p-value** | **OR_{adj}** | **95% CI** | **p-value** |
| Age | - | - | - | 1.2 | 1.0-1.4 | 0.010 |
| Diabetes mellitus | - | - | - | 167.0 | 1.8-15825 | 0.028 |
| DRAGON score | - | - | - | - | - | - |
| Gelsolin | - | - | - | - | - | - |
| DPYSL2 | - | - | - | - | - | - |
| CysA | - | - | - | - | - | - |
| Gelsolin + DPYSL2 | 6.1 | 1.6-22.7 | 0.007 | 14.2 | 2.1-98 | 0.007 |
| Gelsolin + CysA | - | - | - | - | - | - |
| DPYSL2 + CysA | - | - | - | - | - | - |
| Gelsolin + DPYSL2 + CysA | - | - | - | - | - | - |

| **Independent predictor disability at 3^{rd} month** | **Gelsolin + CysA** | | | **Clinical + Gelsolin + CysA** | | |
|---|---|---|---|---|---|---|
| | **OR** | **95% CI** | **p-value** | **OR_{adj}** | **95% CI** | **p-value** |
| Age | - | - | - | **1.1** | 1.0-1.2 | 0.047 |
| Diabetes mellitus | - | - | - | 13.1 | 1.0-173.3 | 0.051 |
| DRAGON score | - | - | - | - | - | - |
| Gelsolin | - | - | - | - | - | - |
| DPYSL2 | - | - | - | - | - | - |
| CysA | - | - | - | - | - | - |
| Gelsolin + DPYSL2 | - | - | - | - | - | - |
| Gelsolin + CysA | 15.1 | 3.7-61.3 | <0.001 | 13.9 | 2.4-80.9 | 0.003 |
| DPYSL2 + CysA | - | - | - | - | - | - |
| Gelsolin + DPYSL2 + CysA | - | - | - | - | - | - |

| **Independent predictor disability at 3^{rd} month** | **Gelsolin + DPYSL2 + CysA** | | | **Clinical + biomarkers combination** | | |
|---|---|---|---|---|---|---|
| | **OR** | **95% CI** | **p-value** | **OR_{adj}** | **95% CI** | **p-value** |
| Age | - | - | - | 1.2 | 1.0-1.4 | 0.025 |
| Diabetes mellitus | - | - | - | 220.0 | 1.2-40603 | 0.043 |
| DRAGON score | - | - | - | - | - | - |
| Gelsolin | - | - | - | - | - | - |
| DPYSL2 | - | - | - | - | - | - |
| CysA | - | - | - | - | - | - |
| Gelsolin + DPYSL2 | - | - | - | - | - | - |
| Gelsolin + CysA | - | - | - | - | - | - |
| DPYSL2 + CysA | - | - | - | - | - | - |
| Gelsolin + DPYSL2 + CysA | 14.5 | 2.8-76.2 | 0.002 | 31.2 | 2.7-359 | 0.006 |

| **Independent predictor disability at 3^{rd} month** | **DRAGON + Gelsolin + DPYSL2** | | | **DRAGON + Gelsolin + CysA** | | |
|---|---|---|---|---|---|---|
| | | **95% CI** | **p-value** | **OR** | **95% CI** | **p-value** |
| Age | - | - | - | - | - | - |
| Diabetes mellitus | - | - | - | - | - | - |
| DRAGON score | 2.2 | 1.1-4.7 | 0.032 | 2.1 | 1.0-4.1 | 0.040 |
| Gelsolin | - | - | - | - | - | - |
| DPYSL2 | - | - | - | - | - | - |
| CysA | - | - | - | - | - | - |
| Gelsolin + DPYSL2 | 7.8 | 1.4-42.3 | 0.017 | - | - | - |
| Gelsolin + CysA | - | - | - | 12.9 | 2.5-67.6 | 0.002 |
| DPYSL2 + CysA | - | - | - | - | - | - |
| Gelsolin + DPYSL2 + CysA | - | - | - | - | - | - |

**Table 4. Independent predictors of long-term outcome for logistic regression models.**

| **Independent predictor disability at 3^{rd} month** | **DRAGON + 3 biomarkers combination** | | |
|---|---|---|---|
| | **OR** | **95% CI** | **p-value** |
| Age | - | - | - |
| Diabetes mellitus | - | - | - |
| DRAGON score | 2.3 | 1.0-5.5 | 0.054 |
| Gelsolin | - | - | - |
| DPYSL2 | - | - | - |
| CysA | - | - | - |
| Gelsolin + DPYSL2 | - | - | - |
| Gelsolin + CysA | - | - | - |
| DPYSL2 + CysA | - | - | - |
| Gelsolin + DPYSL2 + CysA | 30.3 | 2.5-365.4 | 0.007 |

| | | | |
|---|---|---|---|
| Biomarkers were added to clinical model using cut-off point: Gelsolin>19.87 ng/mL, DPYSL2>2095.69 pg/mL (alone) or DPYSL2<2095.69 pg/mL (combination), CysA>31.76 ng/mL. OR_{adj}, odd ratio adjusted by NIHSS and sex; CI, confidence interval. | | | |

As shown in Figure 4A the presence of the biomarkers within the predictive model gradually improves the area under the curve (AUC) of the clinical data from 0.839 to 0.957. Furthermore the use of biomarkers combinations enhanced the discrimination of the model (measured by IDI index), mainly identifying better the non-events (i.e. patients with a mRS at 3rd month <2) (Figure 5A) and allowed a better reclassification of patients into risk categories (by NRI analysis) till 85.7% reclassification (Figure 5B) (Table 5).

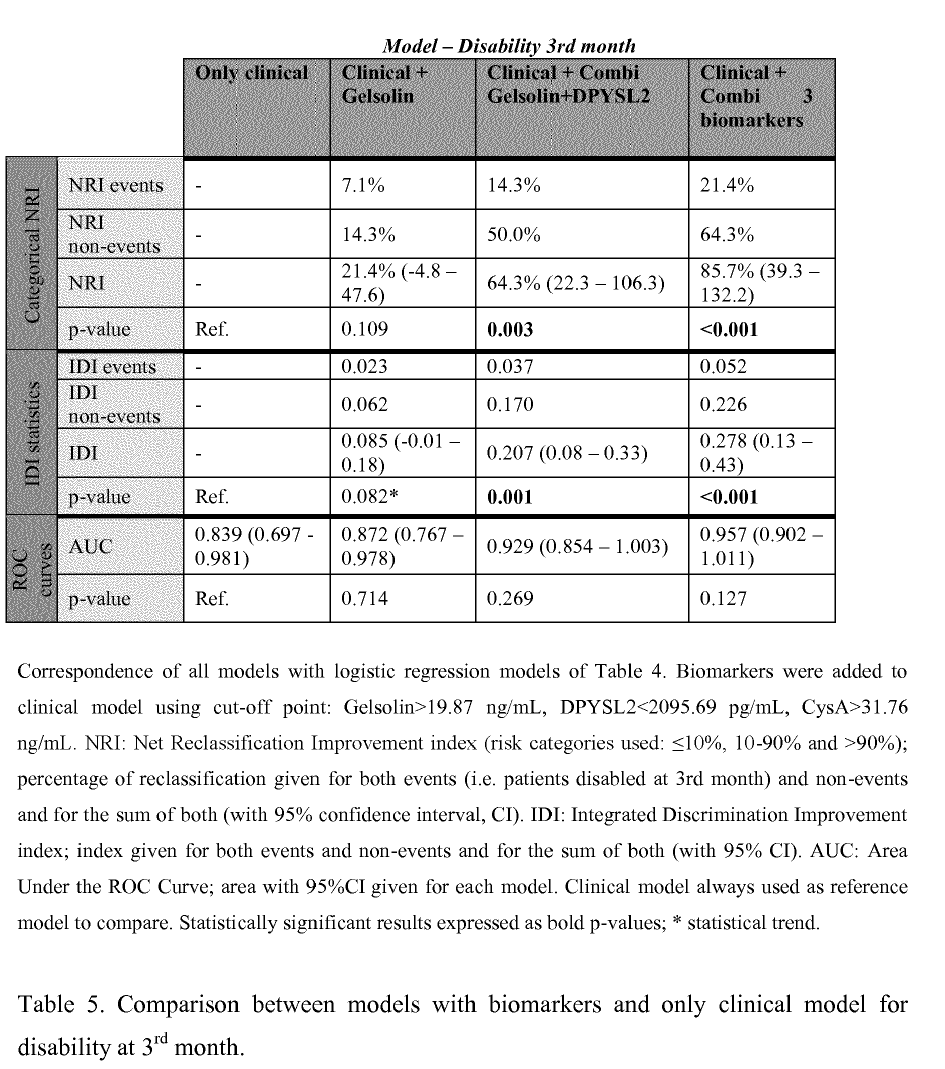

The added value of gelsolin, DPYSL2 and CysA in a validated prediction score of 3rd month functional outcome was also analyzed (DRAGON score; Strbian, D; Neurology, 2012, 78, 427-32). it was found that the biomarkers still add predictive value to this clinical model (Table 6).

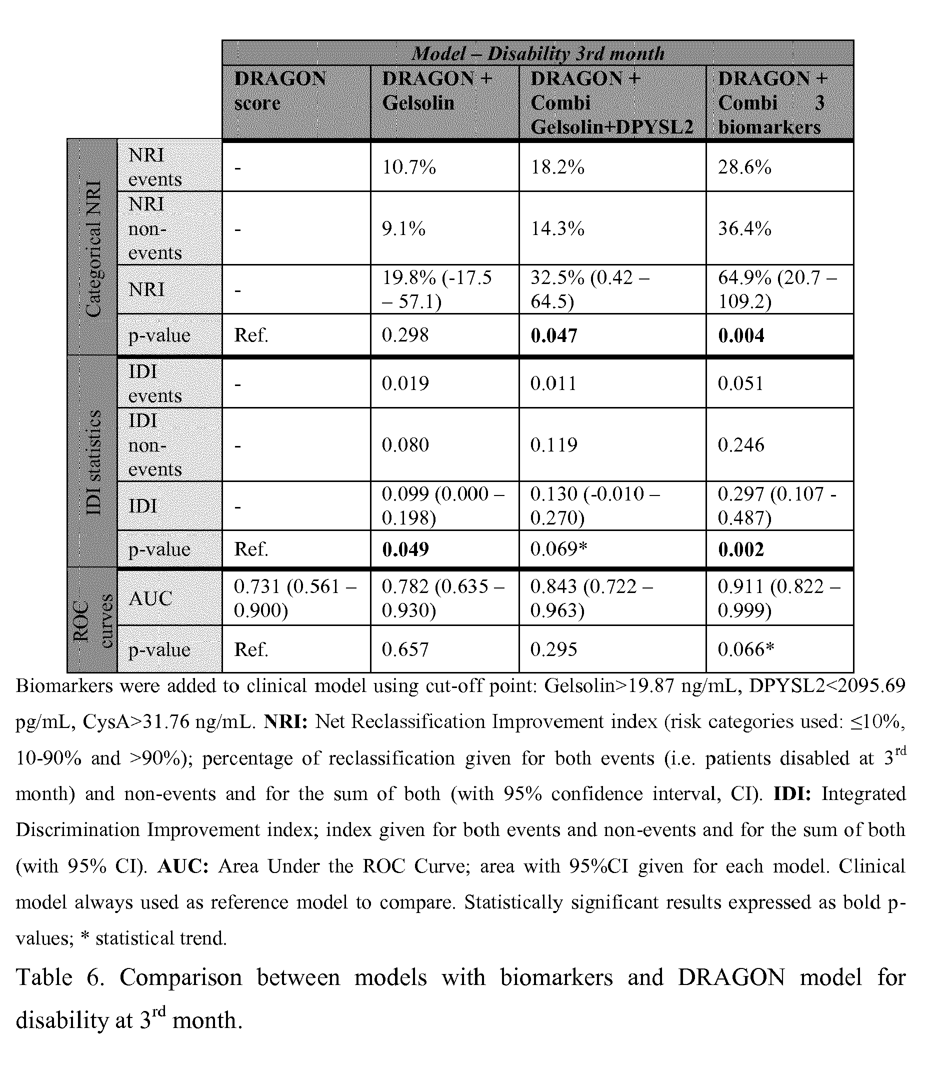

### Example 5- In-hospital mortality

Those patients that die (N = 8, 13.3%) had higher NIHSS score and glycemia level at admission (p = 0.002 and p=0.038, respectively) and had suffered more frequently previous stroke (p=0.071) (Table 3).

CysA showed a trend of being associated with mortality (p = 0.112) (Table 2). A cut-off point for CysA of 34.35 ng/mL (62.5% sensibility, 96% specificity) was associated to in-hospital mortality (Table 3).

The multivariate logistic regression model adjusted by age and sex confirmed NIHSS score at admission (ORadj 1.4 [95% CI 1.1 - 1.9], p = 0.018) and history of previous stroke (ORadj 25.9, [95% CI 1.3 - 512], p = 0.032) as independent predictors of death. When baseline CysA >34.35 ng/mL was added to the clinical model was also an independent predictor (ORadj 316.3, [95% CI 2.17 - 46067], p = 0.024).

The model with CysA improves the AUC of the clinical data from 0.888 to 0.970 (Figure 5B). Moreover it enhanced the discrimination of the model (measured by IDI index) by identifying the events (i.e. patients who died) (Figure 6A) and reclassified a 39% of patients into risk categories (by NRI analysis) (Figure 6B) (Table 7).

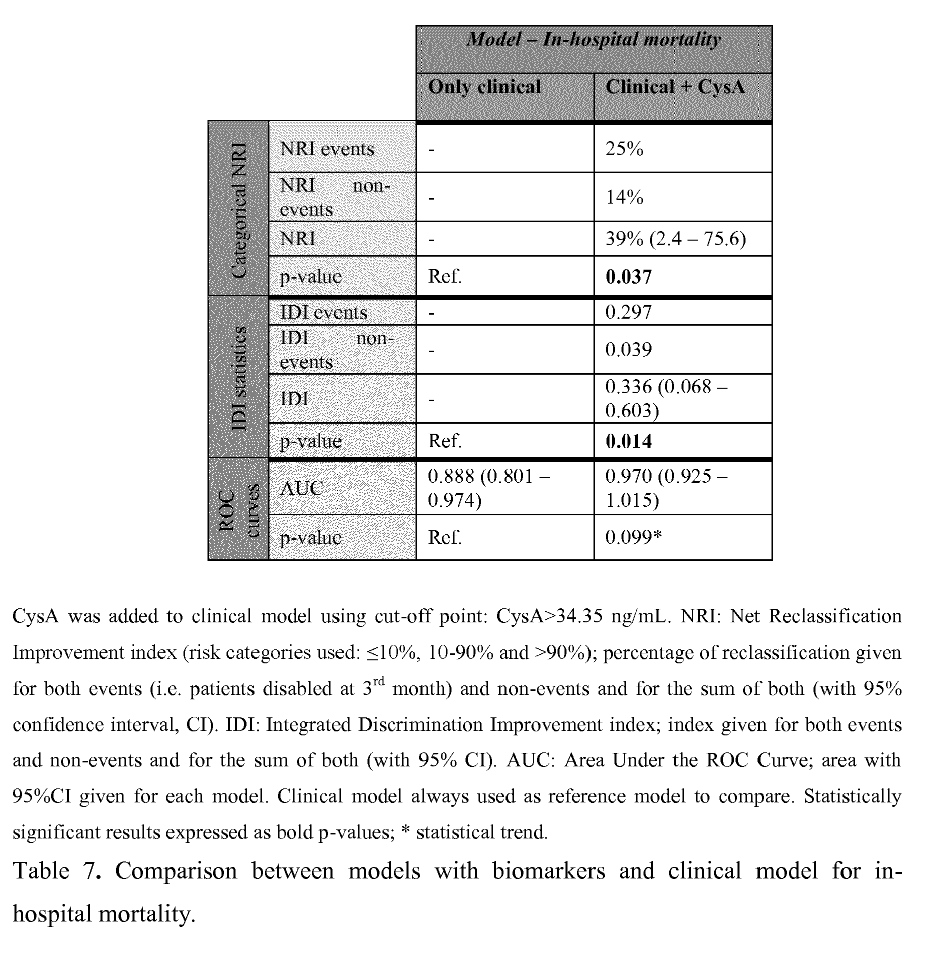

### Example 6- Diagnostic of silent cerebral infarcts

In accordance with results obtained for both gelsolin and DPYSL2 in 60 stroke patients regarding the presence of a previous stroke (Table 2; Figure 7A), ELISAs for both biomarkers were performed in 36 hypertensive subjects, 52.8% of whom had silent brain infarcts.

Those individuals with silent brain infarcts had higher levels of DPYSL2 than subjects without any cerebral event (470.3 [IQR 314.6 - 878.5] vs. 297.8 [IQR 213.8 - 453.8], p = 0.047) (Figure 7B).

## Claims

1. An *in vitro* method for determining the clinical outcome of a patient suffering ischemic stroke, comprising
a) determining the level of a marker selected from gelsolin, CysA, DPYSL2 or a combination thereof, in a sample from said patient, and
b) comparing said level with a reference value for each marker,
wherein if the level of gelsolin or CysA in a sample from said patient is higher than a reference value for each marker and/or if the level of DPYSL2 in a sample from said patient is lower than a reference value for said marker is indicative of a negative clinical outcome
or
wherein if the level of gelsolin or CysA in a sample from said patient is lower than a reference value for each marker and/or if the level of DPYSL2 in a sample from said patient is higher than a reference value for said marker is indicative of a positive clinical outcome.

2. An *in vitro* method for designing an individual therapy for a subject suffering ischemic stroke comprising
a) determining the level of a marker selected from gelsolin, CysA, DPYSL2 or a combination thereof, in a sample from said patient,and
b) comparing said level with a reference value for each marker,
wherein if the level of gelsolin or CysA in a sample from said patient is higher than a reference value for each marker and/or if the level of DPYSL2 in a sample from said patient is lower than the reference value, is indicative that the patient is a candidate for a therapy in a specialized stroke unit.

3. Method according to any of claim 1 or 2 comprising determining the level of gelsolin, CysA and DPYSL2.

4. Method according to any of claims 1 to 3 wherein the patient has been treated with a thrombolytic agent.

5. Method according to any of claims 1 or 3 to 4 wherein the clinical outcome is determined as in-hospital mortality or as the presence of functionally disability at third month after stroke.

6. Method according to any of claims 1 to 5 wherein the reference value of gelsolin is about 19.87 ng protein/ml, the reference value of CysA is about 31.76 ng protein/ml and/or the reference value of DPYSL2 is about 2095.69 pg protein/ml.

7. Method according to any of claims 1 or 3 to 6 wherein if the negative clinical outcome is in-hospital mortality the reference value of CysA is about 34.35 ng protein/ml.

8. Method according to any of claims 1 to 7 further comprising determining one or more clinical parameter, preferably said clinical parameter is selected from the group consisting of age, NIHSS score, presence of diabetes mellitus or previous stroke, sex and DRAGON Score, wherein old age, higher NIHSS score, suffering diabetes mellitus, being woman and/or a DRAGON score is higher than 3 is/are indicative of a negative clinical outcome or indicative that the patient is a candidate for a therapy in a specialized stroke unit.

9. An *in vitro* method for diagnosing a silent cerebrovascular disease comprising:
a) determining the level of a marker selected from gelsolin or DPYSL2 or a combination thereof, in a sample from said patient, and
b) comparing said level with a reference value for each marker,
wherein if the level of DPYSL2 in a sample from said patient is higher than a reference value said marker or if the level of gelsolin in a sample from said patient is lower than a reference value for said marker is indicative that the patient has a silent cerebrovascular disease.

10. Method according to claim 9 wherein the patient is selected from a healthy subject or a subject having vascular risk factors.

11. Method according to any of claims 1 to 10 wherein the sample is a biofluid, preferably blood, plasma, serum, saliva, urine or cerebrospinal fluid.

12. Method according to any of claims 1 to 11 wherein the levels of markers are determined by an immunological technique.

13. A kit comprising a reagent for detecting the level of a marker selected from gelsolin, CysA, DPYSL2 or a combination thereof.

14. A kit according to claim 13 wherein the reagent is an antibody.

15. Use of a kit according to claims 13 or 14 for determining the clinical outcome of a patient suffering ischemic stroke, for designing an individual therapy for a subject suffering ischemic stroke or for diagnosing a silent cerebrovascular disease.
